# EUROPEAN PATENT SPECIFICATION

(11) **EP 2 352 748 B1**
(45) Date of publication and mention of the grant of the patent: **05.03.2014**
(21) Application number: 09736723.9
(22) Date of filing: 25.09.2009
(51) Int. Cl.: C07K 14/415, C12N 15/82

(54) **NITROGEN RESPONSIVE EARLY NODULIN GENE**
AUF STICKSTOFF ANSPRECHENDES FRÜHES NODULINGEN
GÈNE DE NODULINE PRÉCOCE SENSIBLE À L'AZOTE

(30) Priority: 25.09.2008 US 99954 P
(43) Date of publication of application: 10.08.2011
(73) Proprietor: UNIVERSITY OF GUELPH, Guelph, Ontario N1G 2W1 (CA); Syngenta Participations AG, 4058 Basel (CH)
(72) Inventor: KANT, Surya, Guelph, Ontario N1G 1T8 (CA); ROTHSTEIN, Steven, Guelph, Ontario N1G 1K4 (CA); BI, Yong-Mei, Guelph, Ontario N1G 4N3 (CA); CLARKE, Joseph, Durham, North Carolina 27707 (US)
(74) Representative: Evenson, Jane Harriet
(86) International application number: PCT/US2009/058333
(87) International publication number: WO 2010/036866

(56) References cited:
- WO-A1-00/44221
- US-A- 5 631 358
- DATABASE EMBL [Online] 22 September 2004 (2004-09-22), "OsIFCC009393 Oryza sativa Express Library Oryza sativa (indica cultivar-group) genomic, genomic survey sequence." XP002560448 retrieved from EBI accession no. EMBL:CL963400 Database accession no. CL963400
- DATABASE UniProt [Online] 20 March 2007 (2007-03-20), "SubName: Full=Putative uncharacterized protein;" XP002560449 retrieved from EBI accession no. UNIPROT:A2Y947 Database accession no. A2Y947 -& DATABASE EMBL [Online] 20 March 2007 (2007-03-20), XP002560450 retrieved from EBI Database accession no. EAY99607
- XINGMING LIAN ET AL: "Expression Profiles of 10,422 Genes at Early Stage of Low Nitrogen Stress in Rice Assayed using a cDNA Microarray" PLANT MOLECULAR BIOLOGY, KLUWER ACADEMIC PUBLISHERS, DORDRECHT, NL, vol. 60, no. 5, 1 March 2006 (2006-03-01), pages 617-631, XP019262857 ISSN: 1573-5028 cited in the application
- MINGSHENG PENG ET AL: "Genome-wide analysis of Arabidopsis responsive transcriptome to nitrogen limitation and its regulation by the ubiquitin ligase gene NLA" PLANT MOLECULAR BIOLOGY, KLUWER ACADEMIC PUBLISHERS, DORDRECHT, NL, vol. 65, no. 6, 21 September 2007 (2007-09-21), pages 775-797, XP019556096 ISSN: 1573-5028 cited in the application
- YOU M K ET AL: "Identification of genes possibly related to storage root induction in sweetpotato" FEBS LETTERS, ELSEVIER, AMSTERDAM, NL, vol. 536, no. 1-3, 11 February 2003 (2003-02-11), pages 101-105, XP004630019 ISSN: 0014-5793
- KOUCHI HIROSHI ET AL: "Rice ENOD40: Isolation and expression analysis in rice and transgenic soybean root nodules" PLANT JOURNAL, vol. 18, no. 2, April 1999 (1999-04), pages 121-129, XP002560451 ISSN: 0960-7412
- BI YONG-MEI ET AL: "Increased nitrogen-use efficiency in transgenic rice plants over-expressing a nitrogen-responsive early nodulin gene identified from rice expression profiling." PLANT, CELL & ENVIRONMENT DEC 2009, vol. 32, no. 12, December 2009 (2009-12), pages 1749-1760, XP002560452 ISSN: 1365-3040

### SEQUENCE LISTING

<110> UNIVERSITY OF GUELPH
   SYNGENTA PARTICIPATIONS AG
   Bi, Yong-Mei
   Rothstein, Steven
   Kant, Surya
   Clarke, Joseph
<120> NITROGEN RESPONSIVE EARLY NODULIN GENE
<130> 072377-0382384
<160> 3
<170> PatentIn version 3.5
<210> 1
   <211> 575
   <212> DNA
   <213> Oryza sativa Japonica cv. Donjin
<400> 1
   atgtcgacga cggtgacccg tgctcacctg gagcagaggc tcgccctcgc caagcgctgc    60
   tcccggggta cgtacgcacg cacactaaac caccatgttt aatctcgctt tgtcttgcat   120
   tccatggcgc taataactga atatatacaa actgatgtat gcttcgtcga tctgcagagg   180
   cgaaccttgc aggggttaag gcggctgctg tcgcgaccat cgcctctgcc gttccaaccg   240
   tgagtgtgct cagatgctac tagcttcttc taattcctcc ttgtgttgat tcttcagcag   300
   agagtaggtg tactaactta ttagctgcat atgtgtgttg tttcatcgat cagctggcga   360
   gcgtgaggat gctgccatgg gcgaaggcca acatcaaccc gacggggcag gcgctgatca   420
   tctgcacggc ggccgggatg gcatacttcg tcgccgccga caagaagatc ctctcgctgg   480
   ccaggaggca ctccttcgag aacgcacctg aacacctcaa gaacacctcc ttccagggca   540
   ccggccgccc tcaccctgcc ttcttcaggc catga                               575
<210> 2
   <211> 351
   <212> DNA
   <213> Oryza sativa Japonica cv. Donjin
<400> 2
   atgtcgacga cggtgacccg tgctcacctg gagcagaggc tcgccctcgc caagcgctgc    60
   tcccgggagg cgaaccttgc aggggttaag gcggctgctg tcgcgaccat cgcctctgcc   120
   gttccaaccc tggcgagcgt gaggatgctg ccatgggcga aggccaacat caacccgacg   180
   gggcaggcgc tgatcatctg cacggcggcc gggatggcat acttcgtcgc cgccgacaag   240
   aagatcctct cgctggccag gaggcactcc ttcgagaacg cacctgaaca cctcaagaac   300
   acctccttcc agggcaccgg ccgccctcac cctgccttct tcaggccatg a            351
<210> 3
   <211> 116
   <212> PRT
   <213> Oryza sativa Japonica cv. Donjin
<400> 3
   Met Ser Thr Thr Val Thr Arg Ala His Leu Glu Gln Arg Leu Ala Leu
   1               5                   10                  15
   Ala Lys Arg Cys Ser Arg Glu Ala Asn Leu Ala Gly Val Lys Ala Ala
               20                  25                  30
   Ala Val Ala Thr Ile Ala Ser Ala Val Pro Thr Leu Ala Ser Val Arg
           35                  40                  45
   Met Leu Pro Trp Ala Lys Ala Asn Ile Asn Pro Thr Gly Gln Ala Leu
       50                  55                  60
   Ile Ile Cys Thr Ala Ala Gly Met Ala Tyr Phe Val Ala Ala Asp Lys
   65                  70                  75                  80
   Lys Ile Leu Ser Leu Ala Arg Arg His Ser Phe Glu Asn Ala Pro Glu
                   85                  90                  95
   His Leu Lys Asn Thr Ser Phe Gln Gly Thr Gly Arg Pro His Pro Ala
               100                 105                 110
   Phe Phe Arg Pro
           115

## Description

### CROSS-REFERENCE TO RELATED APPLICATIONS

Priority is claimed to U.S. Prov. Patent Appl. No. 61/099,954, filed September 25, 2008, which is incorporated herein in its entirety.

### FIELD OF THE INVENTION

The present invention generally relates to methods for modulating plant characteristics such as nitrogen use efficiency (NUE).

### BACKGROUND OF THE INVENTION

Within the next 50 years, considerable stress will be placed on world-wide crop production from a combination of factors including an increased human population, an increase in the crops used per person, and a number of environmental issues. Given current trends, it will be necessary to approximately double yields world-wide during this time period and it will take a considerable effort to meet this challenge in an economically and environmentally sustainable fashion (Rothstein SJ Plant Cell 2007, 19: 2695-2699). Nitrogen is an essential element for plant growth and is considered to be the main controlling factor for plant productivity after water deficiency (Lea PJ, Morot-Gaudry J-F Plant nitrogen, 2001, Springer, Berlin; London). The production of high-yielding crops is associated with the application of a large quantity of nitrogen fertilizers. These large nitrogen inputs are currently estimated at 90 million metric tons, and have become the major cost in crop production worldwide (Frink CR, et al., PNAS 1999, 96: 1175-1180). However, nitrogen incorporated into agricultural crops rarely exceeds 40% of the applied nitrogen, indicating a serious inefficiency in nitrogen utilization (Raun WR, Johnson GV Agronomy Journal 1999, 91: 357-363; Glass ADM Critical Reviews in Plant Sciences 2003, 22: 453-470). Therefore, there is an urgent need to develop crops with improved nitrogen use efficiency (NUE).

There are two main stages of nitrogen use in the plant life cycle. The first one is the vegetative stage, when nitrogen assimilation is most involved. The second one is the reproductive stage, when both nitrogen assimilation and remobilization are involved. In measuring NUE, several definitions and evaluation methods have been developed over the years, one of those being biomass or grain yield versus the nitrogen amount supplied (Good AG, et al., Trends Plant Sci 2004, 9: 597-605). Generally, this NUE can be divided further into two parts, one being assimilation efficiency, and the other being utilization efficiency.

To assess the molecular basis of plant responses to nitrogen and to identify nitrogen-responsive genes, several studies of nitrogen responses have employed microarray gene expression profiling in *Arabidopsis* (Wang R, et al., Plant Cell 2000, 12: 1491-1509; Wang RC, et al., Plant Physiology 2003, 132: 556-567; Price J, et al., Plant Cell 2004, 16: 2128-2150; Scheible WR, et al., Plant Physiology 2004, 136: 2483-2499). Other studies have investigated nitrogen-responsive genes for *Arabidopsis* under chronic nitrogen stress, as well as the transient transcriptional change after short and long-term nitrogen availability increase (Bi YM, et al., BMC Genomics 2007, 8: 281). Also, a defined nitrogen soil growth system for *Arabidopsis* (Bi YM, et al., Plant Journal 2005, 44: 680-692), was used to isolate the *NLA* gene, which controls the adaptability of *Arabidopsis* to nitrogen limitation, and to identify additional genes involved in nitrogen response (Peng M, et al., Plant J 2007, 50: 320-337; Peng MS, et al., Plant Molecular Biology 2007, 65: 775-797). Studies on nitrogen-responsive genes have also been performed in tomato roots (Wang YH, et al., Plant Physiol 2001, 127: 345-359) and in rice seedlings (Lian X, et al., Plant Mol Biol 2006, 60: 617-631).

Although many nitrogen-responsive genes have been identified from the above studies, reports as to how use this knowledge to manipulate gene expression to enable plants to use nitrogen more efficiently have been lacking. To meet this need, the present disclosure provides a nitrogen responsive early nodulin gene (LOC_Os06g05010), designated *OsENOD93,* and plants that overexpress this nitrogen responsive early nodulin gene, which show increased NUE, including increased seed yield, stress tolerance, nitrate levels and amino acid levels. Also provided are methods for making such plants, or alternatively, methods for mimicking the overexpressing phenotype using an activator of the nitrogen responsive early nodulin gene. Still further provided are isolated nucleic acids and proteins useful in the disclosed methods.

### SUMMARY OF THE INVENTION

The invention is as described in the appended claims. The present disclosure provides isolated OsENOD93 nucleic acids and proteins, vectors and cells expressing the disclosed nucleic acids, and antibodies that specifically bind to the disclosed protein. Representative *OsENOD93* nucleic acids of the invention include nucleic acids comprising (a) a nucleotide sequence set forth as SEQ ID NO:1; (b) a nucleic acid that specifically hybridizes to the complement of SEQ ID NO:1 under stringent hybridization conditions; (c) a nucleotide sequence set forth as SEQ ID NO:2; (d) a nucleic acid that specifically hybridizes to the complement of SEQ ID NO:2 under stringent hybridization conditions; (e) a nucleotide sequence encoding an OsENOD93 protein comprising an amino acid sequence set forth as SEQ ID NO:3. Additionally, representative *OsENOD93* nucleic acids of the disclosure include nucleic acids comprising (a) a nucleotide sequence that is at least 80% identical to SEQ ID NO:1; (b) a nucleotide sequence that is at least 80% identical to SEQ ID NO:2; and, (c) a nucleotide sequence encoding an OsENOD93 protein comprising an amino acid sequence that is at least 80% identical to SEQ ID NO:3. Representative OsENOD93 proteins of the disclosure include proteins comprising (a) an amino acid sequence at least 80% identical to SEQ ID NO:3; and (b) an amino acid sequence of SEQ ID NO:3.

Also provided are *OsENOD93* overexpressing plants, including monocot and dicot plants and methods of producing such plants using the nucleotide sequences of the *OsENOD93* gene disclosed herein. The *OsENOD93* plants are characterized by increased expression of *OsENOD93* and increased nitrogen use efficiency.

Further provided are methods of identifying OsENOD93 binding agents and activators. In one aspect of the disclosure, the method can comprise (a) providing an OsENOD93 protein; (b) contacting the OsENOD93 protein with one or more test agents or a control agent under conditions sufficient for binding; (c) assaying binding of a test agent to the isolated OsENOD93 protein; and (d) selecting a test agent that demonstrates specific binding to the OsENOD93 protein. In another aspect of the disclosure, the method can comprise (a) providing a cell expressing an OsENOD93 protein; (b) contacting the cell with one or more test agents or a control agent; (c) assaying expression of the OsENOD93 gene; and (d) selecting a test agent that induces elevated expression of the gene when contacted with the test agent as compared to the control agent. In another aspect of the disclosure, the method can comprise (a) providing a plant expressing an OsENOD93 protein; (b) contacting the plant with one or more test agents or a control agent; (c) assaying nitrate content, nitrogen uptake, lateral root growth, amino acid content, seed yield, or biomass of the plant; and (d) selecting a test agent that induces increased nitrate content, increased nitrate uptake, increased lateral root growth, seed yield, and/or increased biomass when contacted with the test agent as compared to the control agent.

Still further provided are methods of identifying enhancers of OsENOD93 in a plant comprising (a) providing a cell expressing an OsENOD93 protein; (b) contacting the cell with one or more test agents or a control agent; (c) assaying expression of an OsENOD93 target gene; and (d) selecting a test agent that induces elevated expression of the gene, which gene is normally subject to OsENOD93 induction, when contacted with the test agent as compared to the control agent. In another aspect of the disclosure, the method can comprise (a) providing a plant expressing an OsENOD93 protein; (b) contacting the plant with one or more test agents or a control agent; (c) assaying nitrate content, nitrogen uptake, lateral root growth, amino acid content, seed yield, or biomass of the plant; and (d) selecting a test agent that induces increased nitrate content, increased nitrate uptake, increased lateral root growth, seed yield, and/or increased biomass when contacted with the test agent as compared to the control agent.

Methods are also provided for improving plant yield using OsENOD93 binding agents and activators identified according to the methods disclosed herein.

### BRIEF DESCRIPTION OF THE DRAWINGS

Figure 1A shows the mean shoot dry weight (± SD, n=3-5) in wild type plants grown in 1mM, 5mM and 10mM nitrate for 28 days.
Figure 1B shows the mean root dry weight (± SD, n=3-5) in wild type plants grown in 1mM, 5mM and 10mM nitrate for 28 days.
Figure 1C shows the mean shoot to root ratio (± SD, n=3-5) as measured by dry weight in wild type plants grown in 1mM, 5mM and 10mM nitrate for 28 days.
Figure 1D shows the mean chlorophyll content (± SD, n=3-5), expressed as mg/g (fresh weight) of plant, in wild type plants grown in 1mM, 5mM and 10mM nitrate for 28 days.
Figure 1E shows the mean anthocyanin content (± SD, n=3-5), expressed as units/g (fresh weight) of plant, in wild type plants grown in 1mM, 5mM and 10mM nitrate for 28 days.
Figure 2A shows the mean nitrate content in shoots (± SD, n=3-5), expressed as mg/g (fresh weight) of plant, in wild type plants grown in 1mM, 5mM and 10mM nitrate for 28 days.
Figure 2B shows the mean nitrate content in roots (± SD, n=3-5), expressed as mg/g (fresh weight) of plant, in wild type plants grown in 1mM, 5mM and 10mM nitrate for 28 days.
Figure 2C shows the mean total amino acids in shoots (± SD, n=3-5), expressed as micromole mol/g (fresh weight) of plant, in wild type plants grown in 1mM, 5mM and 10mM nitrate for 28 days.
Figure 2D shows the mean total amino acids in roots (± SD, n=3-5), expressed as micromole mol/g (fresh weight) of plant, in wild type plants grown in 1mM, 5mM and 10mM nitrate for 28 days.
Figure 2E shows the mean total soluble protein in shoots (± SD, n=3-5), expressed as mg/g (fresh weight) of plant, in wild type plants grown in 1mM, 5mM and 10mM nitrate for 28 days.
Figure 2F shows the mean total soluble protein in roots (± SD, n=3-5), expressed as mg/g (fresh weight) of plant, in wild type plants grown in 1mM, 5mM and 10mM nitrate for 28 days.
Figure 2G shows the mean nitrogen content in shoots (± SD, n=3-5), expressed as percentage of dry weight of plant, in wild type plants grown in 1mM, 5mM and 10mM nitrate for 28 days.
Figure 2H shows the mean nitrogen content in roots (± SD, n=3-5), expressed as percentage of dry weight of plant, in wild type plants grown in 1mM, 5mM and 10mM nitrate for 28 days.
Figure 3A shows the mean nitrate content in shoots (± SD, n=3-5), expressed as mg/g (fresh weight) of plant, in wild type plants grown in 1mM nitrate for 28 days and plants grown in 1mM nitrate for 28 days followed by 2 hours growth in 10mM nitrate (induction).
Figure 3B shows the mean nitrate content in roots (± SD, n=3-5), expressed as mg/g (fresh weight) of plant, in wild type plants grown in 1mM nitrate for 28 days and plants grown in 1mM nitrate for 28 days followed by 2 hours growth in 10mM nitrate (induction).
Figure 3C shows the mean nitrate content in shoots (± SD, n=3-5), expressed as mg/g (fresh weight) of plant, in wild type plants grown in 10mM nitrate for 28 days and plants grown in 10mM nitrate for 28 days followed by 2 hours growth in 1mM nitrate (reduction).
Figure 3D shows the mean nitrate content in roots (± SD, n=3-5), expressed as mg/g (fresh weight) of plant, in wild type plants grown in 10mM nitrate for 28 days and plants grown in 10mM nitrate for 28 days followed by 2 hours growth in 1mM nitrate (reduction).
Figure 4A shows the relative transcript abundance, as determined by microarray expression analysis, of *OsENOD93* in roots of wild type plants grown in 10mM nitrate for 28 days and plants grown in 10mM nitrate for 28 days, 1mM nitrate for 28 days, 10mM nitrate followed by 2 hours growth in 1mM nitrate (reduction) and 1mM nitrate for 28 days followed by 2 hours in 10 mM nitrate.
Figure 4B shows the expression of the *OsENOD93* gene at different growth stages and in different plant parts in wild type rice plants.
Figure 4C shows the relative expression levels of the *OsENOD93* gene in both the shoots and roots of wild type plants grown in 1mM nitrate and 10mM nitrate for 28 days as determined by quantitative real-time PCR using actin as an internal control. Expression levels are normalized to the wild type shoot grown in 1mM nitrate.
Figure 4D shows the relative expression levels of the *OsENOD93* gene in both the shoots and roots of transgenic plants overexpressing the *OsENOD93* gene grown in 1mM nitrate and 10mM nitrate for 28 days as determined by quantitative real-time PCR using actin as an internal control. Expression levels are normalized to the wild type shoot grown in 1mM nitrate.
Figure 5A shows the mean total amino acids in roots (± SD, n=3-5), expressed as micromole mol/g (fresh weight) of plant, in both wild type plants and transgenic plants overexpressing the *OsENOD93* gene grown in 1mM, 3mM and 10mM nitrate for 28 days.
Figure 5B shows the mean nitrogen content in roots (± SD, n=3-5), expressed as percentage of dry weight of plant, in both wild type plants and transgenic plants overexpressing the *OsENOD93* gene grown in 1mM, 3mM and 10mM nitrate for 28 days.
Figure 5C shows the mean total amino acids in roots (± SD, n=3-5), expressed as micromole/g (fresh weight) of plant, in both wild type plants and transgenic plants overexpressing the *OsENOD93* gene grown in 3mM and 10mM nitrate for 60 days.
Figure 5D shows the mean nitrogen content in roots (± SD, n=3-5), expressed as a percentage of dry weight of plant, in both wild type plants and transgenic plants overexpressing the *OsENOD93* gene grown in 3mM and 10mM nitrate for 60 days.
Figure 6A shows the genomic sequence of the *OsENOD93* gene (SEQ ID NO:1).
Figure 6B shows the cDNA sequence of the *OsENOD93* gene (SEQ ID NO:2).
Figure 6C shows the predicted amino acid sequence of the protein encoded by the *OsENOD93* gene (SEQ ID NO:3).
Figure 6D shows the location of three exons (underlined) and two introns within the genomic sequence of the *OsENOD93.*
Figure 6E shows the *OsENOD93* domain (underlined) in the amino acid sequence.
Figure 7 shows the total amino acids in xylem sap (± SD, n=3-5), expressed as micromole/ml of xylem sap, in both wild type and transgenic rice plants overexpressing the *OsENOD93* gene grown in 1mM, 3mM, and 10mM nitrate for 28 days. Bars with different letters indicate significant difference at P<.05 (Fisher's protected least significant difference test).

### DETAILED DESCRIPTION

### I. OsENOD93 nucleic acids and proteins

The present disclosure provides *OsENOD93* nucleic acids and proteins, variants thereof, and activators thereof. Previously described nucleic acids and proteins have not taught how to use such molecules for promoting nitrogen use efficiency in plants, as presently disclosed.

A representative *OsENOD93* nucleic acid is set forth as SEQ ID NO:1, which encodes the representative OsENOD93 protein of SEQ ID NO:3. OsENOD93 variants of the present disclosure include variant proteins having OsENOD93 activity.

In leguminous plants early nodulin *(ENOD)* genes are expressed during nodule development and may mediate nodule organogenesis (Mylona et al., 1995). However differential roles of *ENOD* genes have also been postulated. *ENOD40* has shown to be expressed during early stage of nodule initiation (Kouchi and Hata, 1993; Yang et al., 1993) and its protein might be involved in cortical cell division (Charon et al. 1997), differentiation of vascular bundles (Kouchi et al., 1999) and also possible role in transporting photosynthates (Kouchi and Hata, 1993; Yang et al., 1993). Similarly, expression of *GmENOD93* has been found in soybean nodules (Kouchi and Hata, 1993) and it has a homolog in rice Os*ENOD93a* (Reddy et al., 1998), although its function has not been characterized. A homology search of the OsENOD93 gene of the present disclosure in rice indicates that there is family of at least five genes having an ENOD93 domain and that the *OsENOD93* gene of the present disclosure encodes a protein that has approximately 98% amino acid sequence identity with *OsENOD93a.*

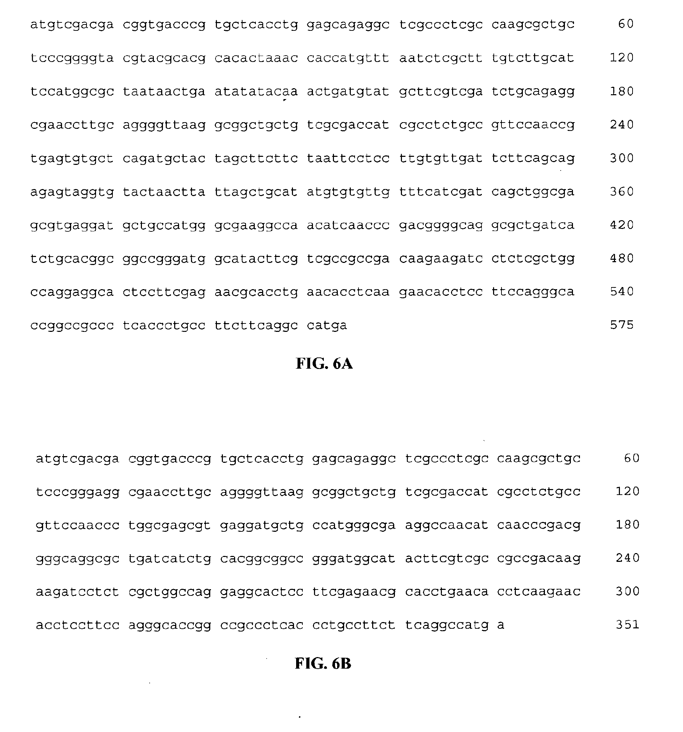

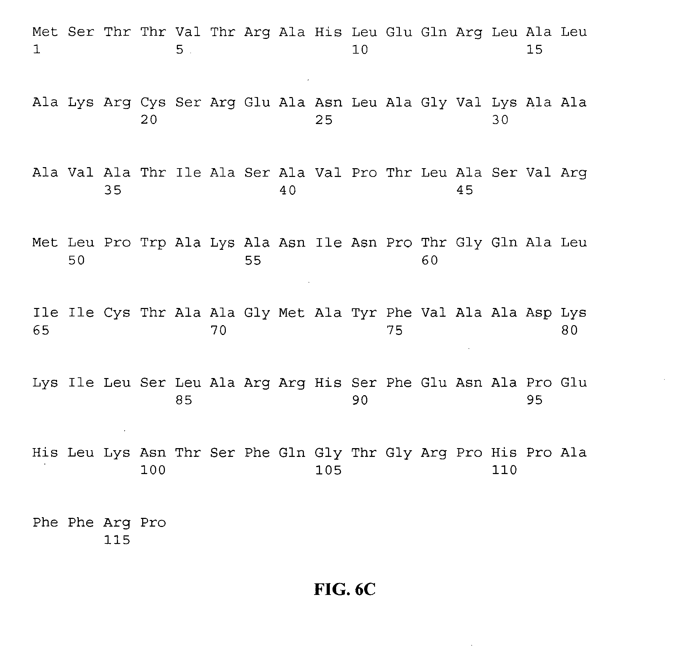

The *OsENOD93* gene of the present disclosure consists of three exons and two introns and encodes a protein of 116 amino acids with a molecular weight 12424 Da and a pI of 10.95 (Figures 6C-E and Table 6).

### I.A. OsENOD93 Nucleic Acids

Nucleic acids are deoxyribonucleotides or ribonucleotides and polymers thereof in single-stranded, double-stranded, or triplexed form. Unless specifically limited, the term encompasses nucleic acids containing known analogues of natural nucleotides that have similar properties as the reference natural nucleic acid. The terms nucleic acid molecule or nucleic acid may also be used in place of gene, cDNA, mRNA, or cRNA. Nucleic acids may be synthesized, or may be derived from any biological source, including any organism.

Representative nucleic acids of the disclosure comprise the nucleotide sequences of SEQ ID NO:1 and SEQ ID NO:2 and substantially identical sequences encoding OsENOD93 proteins with substantially identical activity, for example, sequences at least 50% identical to SEQ ID NO:1, or SEQ ID NO:2, such as at least 55% identical; or at least 60% identical; or at least 65% identical; such as at least 70% identical; or at least 75% identical; or at least 80% identical; or at least 85% identical; or at least 90% identical, or as at least 91% identical; or at least 92% identical; or at least 93% identical; or at least 94% identical; or at least 95% identical; or at least 96% identical; or at least 97% identical; or at least 98% identical; or at least 99% identical. Sequences are compared for maximum correspondence using a sequence comparison algorithm using the full-length sequence of SEQ ID NO:1, or SEQ ID NO:2 as the query sequence, as described herein below, or by visual inspection.

Substantially identical sequences may be polymorphic sequences, i.e, alternative sequences or alleles in a population. An allelic difference may be as small as one base pair.

Substantially identical sequences may also comprise mutagenized sequences, including sequences comprising silent mutations. A mutation may comprise one or more residue changes, a deletion of one or more residues, or an insertion of one or more additional residues.

Substantially identical nucleic acids are also identified as nucleic acids that hybridize specifically to or hybridize substantially to the full length of SEQ ID NO:1, or SEQ ID NO:2 under stringent conditions. In the context of nucleic acid hybridization, two nucleic acid sequences being compared may be designated a probe and a target. A probe is a reference nucleic acid molecule, and a target is a test nucleic acid molecule, often found within a heterogeneous population of nucleic acid molecules. A target sequence is synonymous with a test sequence.

A particular nucleotide sequence employed for hybridization studies or assays includes probe sequences that are complementary to at least an about 14 to 40 nucleotide sequence of a nucleic acid molecule of the present disclosure. Preferably, probes comprise 14 to 20 nucleotides, or even longer where desired, such as 30, 40, 50, 60, 100, 200, 300, or 500 nucleotides or up to the full length of SEQ ID NO:1, or SEQ ID NO:2. Such fragments may be readily prepared, for example by chemical synthesis of the fragment, by application of nucleic acid amplification technology, or by introducing selected sequences into recombinant vectors for recombinant production.

Specific hybridization refers to the binding, duplexing, or hybridizing of a molecule only to a particular nucleotide sequence under stringent conditions when that sequence is present in a complex nucleic acid mixture (e.g., total cellular DNA or RNA). Specific hybridization may accommodate mismatches between the probe and the target sequence depending on the stringency of the hybridization conditions.

Stringent hybridization conditions and stringent hybridization wash conditions in the context of nucleic acid hybridization experiments such as Southern and Northern blot analysis are both sequence- and environment-dependent. Longer sequences hybridize specifically at higher temperatures. An extensive guide to the hybridization of nucleic acids is found in Tijssen, Laboratory Techniques in Biochemistry and Molecular Biology-Hybridization with Nucleic Acid Probes, 1993, part I chapter 2, Elsevier, New York, New York. Generally, highly stringent hybridization and wash conditions are selected to be about 5°C lower than the thermal melting point (Tm) for the specific sequence at a defined ionic strength and pH. Typically, under stringent conditions a probe will hybridize specifically to its target subsequence, but to no other sequences.

The Tm is the temperature (under defined ionic strength and pH) at which 50% of the target sequence hybridizes to a perfectly matched probe. Very stringent conditions are selected to be equal to the Tm for a particular probe. An example of stringent hybridization conditions for Southern or Northern Blot analysis of complementary nucleic acids having more than about 100 complementary residues is overnight hybridization in 50% formamide with 1 mg of heparin at 42°C. An example of highly stringent wash conditions is 15 minutes in 0.1X Saline-Sodium Citrate (SSC) at 65°C. An example of stringent wash conditions is 15 minutes in 0.2X SSC buffer at 65°C. *See* Sambrook et al., eds., Molecular Cloning: A Laboratory Manual, 1989, Cold Spring Harbor Laboratory Press, Cold Spring Harbor, New York, for a description of SSC buffer. Often, a high stringency wash is preceded by a low stringency wash to remove background probe signal. An example of medium stringency wash conditions for a duplex of more than about 100 nucleotides, is 15 minutes in 1X SSC at 45°C. An example of low stringency wash for a duplex of more than about 100 nucleotides, is 15 minutes in 4X to 6X SSC at 40°C. For short probes (e.g., about 10 to 50 nucleotides), stringent conditions typically involve salt concentrations of less than about 1M Na+ ion, typically about 0.01 to 1M Na+ ion concentration (or other salts) at pH 7.0-8.3, and the temperature is typically at least about 30°C. Stringent conditions may also be achieved with the addition of destabilizing agents such as formamide. In general, a signal to noise ratio of 2-fold (or higher) than that observed for an unrelated probe in the particular hybridization assay indicates detection of a specific hybridization.

The following are examples of hybridization and wash conditions that may be used to identify nucleotide sequences that are substantially identical to reference nucleotide sequences of the present disclosure: a probe nucleotide sequence preferably hybridizes to a target nucleotide sequence in 7% sodium dodecyl sulphate (SDS), 0.5M NaP04, 1mM EDTA at 50°C followed by washing in 2X SSC, 0.1% SDS at 50°C; more preferably, a probe and target sequence hybridize in 7% sodium dodecyl sulphate (SDS), 0.5M NaPO4, 1mM EDTA at 50°C followed by washing in 1X SSC, 0.1% SDS at 50°C; more preferably, a probe and target sequence hybridize in 7% sodium dodecyl sulphate (SDS), 0.5M NaPO4, 1mM EDTA at 50°C followed by washing in 0.5X SSC, 0.1% SDS at 50°C; more preferably, a probe and target sequence hybridize in 7% sodium dodecyl sulphate (SDS), 0.5M NaPO4, 1mM EDTA at 50°C followed by washing in 0.1X SSC, 0.1% SDS at 50°C; more preferably, a probe and target sequence hybridize in 7% sodium dodecyl sulphate (SDS), 0.5M NaPO4, 1mM EDTA at 50°C followed by washing in 0.1X SSC, 0.1% SDS at 65°C.

A further indication that two nucleic acid sequences are substantially identical is that proteins encoded by the nucleic acids are substantially identical, share an overall three-dimensional structure, or are biologically functional equivalents. These terms are defined further herein below. Nucleic acid molecules that do not hybridize to each other under stringent conditions are still substantially identical if the corresponding proteins are substantially identical. This may occur, for example, when two nucleotide sequences comprise conservatively substituted variants as permitted by the genetic code.

The term conservatively substituted variants refers to nucleic acid sequences having degenerate codon substitutions wherein the third position of one or more selected (or all) codons is substituted with mixed-base and/or deoxyinosine residues. *See* Batzer et al., Nucleic Acids Res., 1991, 19:5081; Ohtsuka et al., J. Biol. Chem., 1985, 260:2605-2608; and Rossolini et al. Mol. Cell Probes, 1994, 8:91-98.

Nucleic acids of the disclosure also comprise nucleic acids complementary to SEQ ID NO:1 and SEQ ID NO:2. Complementary sequences are two nucleotide sequences that comprise antiparallel nucleotide sequences capable of pairing with one another upon formation of hydrogen bonds between base pairs. As used herein, the term complementary sequences means nucleotide sequences which are substantially complementary, as may be assessed by the same nucleotide comparison methods set forth below, or is defined as being capable of hybridizing to the nucleic acid segment in question under relatively stringent conditions such as those described herein. A particular example of a complementary nucleic acid segment is an antisense oligonucleotide.

Nucleic acids of the disclosure also comprise nucleic acids of SEQ ID NO:1 and SEQ ID NO:2, which have been altered for expression in organisms other than plants to account for differences in codon usage between plants and the other organism. For example, the specific codon usage in plants differs from the specific codon usage in certain microorganisms. Comparison of the usage of codons within a cloned microbial ORF to usage in plant genes (and in particular genes from the target plant) will enable an identification of the codons within the ORF that should specifically be changed. Typically plant evolution has tended towards a strong preference of the nucleotides C and G in the third base position of monocotyledons, whereas dicotyledons often use the nucleotides A or T at this position. By modifying a gene to incorporate specific codon usage for a particular target transgenic species, many of the problems described below for GC/AT content and illegitimate splicing will be overcome.

Plant genes typically have a GC content of more than 35%. ORF sequences which are rich in A and T nucleotides can cause several problems in plants. Firstly, motifs of ATTTA are believed to cause destabilization of messages and are found at the 3' end of many short-lived mRNAs. Secondly, the occurrence of polyadenylation signals such as AATAAA at inappropriate positions within the message is believed to cause premature truncation of transcription. In addition, monocotyledons may recognize AT-rich sequences as splice sites (see below).

The term subsequence refers to a sequence of nucleic acids that comprises a part of a longer nucleic acid sequence. An exemplary subsequence is a probe, described herein above, or a primer. The term primer as used herein refers to a contiguous sequence comprising about 8 or more deoxyribonucleotides or ribonucleotides, preferably 10-20 nucleotides, and more preferably 20-30 nucleotides of a selected nucleic acid molecule. The primers of the disclosure encompass oligonucleotides of sufficient length and appropriate sequence so as to provide initiation of polymerization on a nucleic acid molecule of the present disclosure.

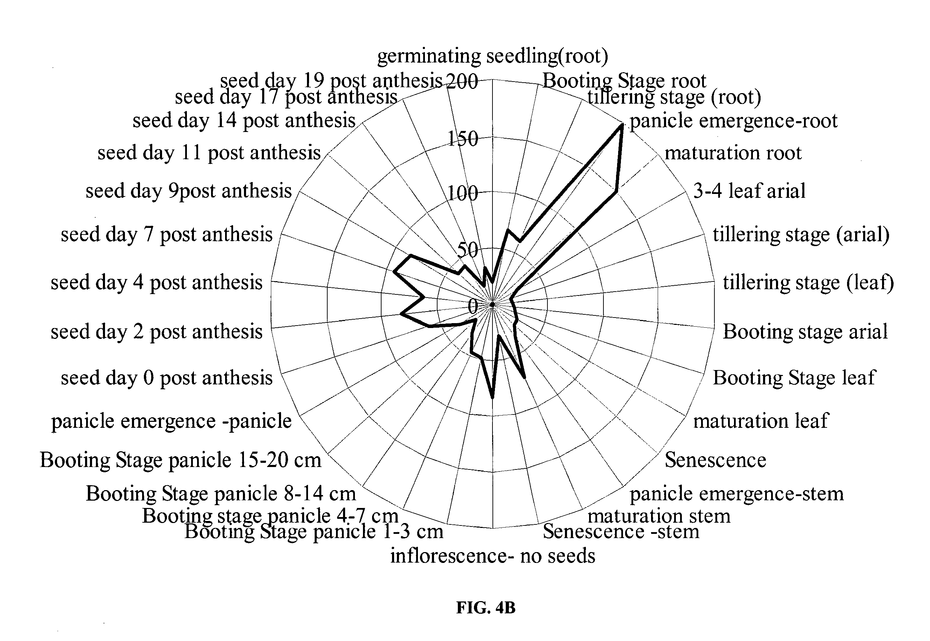

Representative nucleic acids of the disclosure also include nucleic acids consisting essentially of SEQ ID NO:1 and SEQ ID NO:2, in as much as the nucleic acids are isolated from and do not contain additional nucleotide sequences with which the nucleic acids may be normally associated. Additionally, the nucleic acid of the invention may be expressed in the entire plant, or may be differentially expressed in particular parts of the plant. Further, the nucleic acids of the invention may be expressed at different times throughout the development of the plant, such as during the reproductive stage or vegetative stage of plant development (e.g., the developmental stages and plant parts identified in Figure 4B).

Vectors are provided comprising the disclosed nucleic acids, including vectors for recombinant expression, wherein a nucleic acid of the invention is operably linked to a functional promoter. When operably linked to a nucleic acid, a promoter is in functional combination with the nucleic acid such that the transcription of the nucleic acid is controlled and regulated by the promoter region. Vectors refer to nucleic acids capable of replication in a host cell, such as plasmids, cosmids, and viral vectors.

Nucleic acids of the present disclosure may be cloned, synthesized, altered, mutagenized, or combinations thereof. Standard recombinant DNA and molecular cloning techniques used to isolate nucleic acids are known in the art. Site-specific mutagenesis to create base pair changes, deletions, or small insertions is also known in the art. *See e.g.,* Sambrook et al. (eds.) Molecular Cloning: A Laboratory Manual, 1989, Cold Spring Harbor Laboratory Press, Cold Spring Harbor, New York; Silhavy et al., Experiments with Gene Fusions, 1984, Cold Spring Harbor Laboratory Press, Cold Spring Harbor, New York; Glover & Hames, DNA Cloning: A Practical Approach, 2nd ed., 1995, IRL Press at Oxford University Press, Oxford/New York; Ausubel (ed.) Short Protocols in Molecular Biology, 3rd ed., 1995, Wiley, New York.

In another aspect of the disclosure, a method is provided for detecting a nucleic acid molecule that encodes an OsENOD93 protein. Such methods may be used to detect OsENOD93 gene variants or altered gene expression. Sequences detected by methods of the disclosure may be detected, subcloned, sequenced, and further evaluated by any measure well known in the art using any method usually applied to the detection of a specific DNA sequence. Thus, the nucleic acids of the present disclosure may be used to clone genes and genomic DNA comprising the disclosed sequences. Alternatively, the nucleic acids of the present disclosure may be used to clone genes and genomic DNA of related sequences. Levels of an *OsENOD93* nucleic acid molecule may be measured, for example, using an RT-PCR assay. *See* Chiang, J. Chromatogr. A., 1998, 806:209-218, and references cited therein.

In another aspect of the disclosure, genetic assays using *OsENDO93* nucleic acids may be performed for quantitative trait loci (QTL) analysis and to screen for genetic variants, for example by allele-specific oligonucleotide (ASO) probe analysis (Conner et al., Proc. Natl. Acad. Sci. USA, 1983, 80(1):278-282), oligonucleotide ligation assays (OLAs) (Nickerson et al., Proc. Natl. Acad. Sci. USA, 1990, 87(22):8923-8927), single-strand conformation polymorphism (SSCP) analysis (Orita et al., Proc. Natl. Acad . Sci. USA, 1989, 86(8):2766-2770), SSCP/heteroduplex analysis, enzyme mismatch cleavage, direct sequence analysis of amplified exons (Kestila et al., Mol. Cell, 1998, 1(4):575-582; Yuan et al., Hum. Mutat., 1999, 14(5):440-446), allele-specific hybridization (Stoneking et al., Am. J. Hum. Genet., 1991, 48(2):370-382), and restriction analysis of amplified genomic DNA containing the specific mutation. Automated methods may also be applied to large-scale characterization of single nucleotide polymorphisms (Wang et al., Am. J. Physiol., 1998, 274(4 Pt 2):H1132-1140; Brookes, Gene, 1999, 234(2):177-186). Preferred detection methods are non-electrophoretic, including, for example, the TAQMAN™ allelic discrimination assay, PCR-OLA, molecular beacons, padlock probes, and well fluorescence. *See* Landegren et al., Genome Res., 1998, 8:769-776 and references cited therein.

### I.B. OsENOD93 Proteins

The present disclosure also provides isolated OsENOD93 polypeptides. Polypeptides and proteins each refer to a compound made up of a single chain of amino acids joined by peptide bonds. A representative OsENOD93 polypeptide is set forth as SEQ ID NO:3. Additional polypeptides of the disclosure include OsENOD93 proteins with substantially identical activity, for example, sequences at least 50% identical to SEQ ID NO:3, such as at least 55% identical; or at least 60% identical; or at least 65% identical; such as at least 70% identical; or at least 75% identical; or at least 80% identical; or at least 85% identical; or at least 90% identical, or as at least 91% identical; or at least 92% identical; or at least 93% identical; or at least 94% identical; or at least 95% identical; or at least 96% identical; or at least 97% identical; or at least 98% identical; or at least 99% identical. Sequences are compared for maximum correspondence using a sequence comparison algorithm using the full-length sequence of SEQ ID NO:3 as the query sequence, as described herein below, or by visual inspection. Representative proteins of the invention also include polypeptides consisting essentially of SEQ ID NO:3, in as much as the polypeptides are isolated from and do not contain additional amino acid sequences with which the polypeptides may be normally associated. Polypeptides encoded by any one of the nucleic acids of the disclosure are disclosed herein.

Polypeptides of the disclosure may comprise naturally occurring amino acids, synthetic amino acids, genetically encoded amino acids, non-genetically encoded amino acids, and combinations thereof. Polypeptides may include both L-form and D-form amino acids.

Representative non-genetically encoded amino acids include but are not limited to 2-aminoadipic acid; 3-aminoadipic acid; β-aminopropionic acid; 2-aminobutyric acid; 4-aminobutyric acid (piperidinic acid); 6-aminocaproic acid; 2-aminoheptanoic acid; 2-aminoisobutyric acid; 3-aminoisobutyric acid; 2-aminopimelic acid; 2,4-diaminobutyric acid; desmosine; 2,2'-diaminopimelic acid; 2,3-diaminopropionic acid; N-ethylglycine; N-ethylasparagine; hydroxylysine; allo-hydroxylysine; 3-hydroxyproline; 4-hydroxyproline; isodesmosine; allo-isoleucine; N-methylglycine (sarcosine); N-methylisoleucine; N-methylvaline; norvaline; norleucine; and ornithine.

Representative derivatized amino acids include, for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzylhistidine.

The present disclosure also provides functional fragments of an OsENOD93 polypeptide, for example, fragments that have activity similar to that of a full-length OsENOD93 protein. Functional polypeptide sequences that are longer than the disclosed sequences are also provided. For example, one or more amino acids may be added to the N-terminus or C-terminus of a polypeptide. Such additional amino acids may be employed in a variety of applications, including but not limited to purification applications. Methods of preparing elongated proteins are known in the art.

OsENOD93 proteins of the disclosure include proteins comprising amino acids that are conservatively substituted variants of SEQ ID NO:3. A conservatively substituted variant refers to a polypeptide comprising an amino acid in which one or more residues have been conservatively substituted with a functionally similar residue.

Examples of conservative substitutions include the substitution of one non-polar (hydrophobic) residue such as isoleucine, valine, leucine or methionine for another; the substitution of one polar (hydrophilic) residue for another such as between arginine and lysine, between glutamine and asparagine, between glycine and serine; the substitution of one basic residue such as lysine, arginine or histidine for another; or the substitution of one acidic residue, such as aspartic acid or glutamic acid for another.

Isolated polypeptides of the disclosure may be purified and characterized using a variety of standard techniques that are known to the skilled artisan. *See e.g.,* Schröder et al., The Peptides, 1965, Academic Press, New York; Bodanszky, Principles of Peptide Synthesis, 2nd rev. ed. 1993, Springer-Verlag, Berlin/ New York; Ausubel (ed.), Short Protocols in Molecular Biology, 3rd ed., 1995, Wiley, New York.

The present disclosure further provides methods for detecting an OsENOD93 polypeptide. The disclosed methods can be used, for example, to determine altered levels of OsENOD93 protein, for example, induced levels of OsENOD93 protein.

For example, the method may involve performing an immunochemical reaction with an antibody that specifically recognizes an OsENOD93 protein. Techniques for detecting such antibody-antigen conjugates or complexes are known in the art and include but are not limited to centrifugation, affinity chromatography and other immunochemical methods. *See e.g.,* Ishikawa Ultrasensitive and Rapid Enzyme Immunoassay, 1999, Elsevier, Amsterdam/New York, United States of America; Law, Immunoassay: A Practical Guide, 1996, Taylor & Francis, London/Bristol, Pennsylvania, United States of America; Liddell et al., Antibody Technology, 1995, Bios Scientific Publishers, Oxford, United Kingdom; and references cited therein.

### I.C. Nucleotide and Amino Acid Sequence Comparisons

The terms identical or percent identity in the context of two or more nucleotide or protein sequences, refer to two or more sequences or subsequences that are the same or have a specified percentage of amino acid residues or nucleotides that are the same, when compared and aligned for maximum correspondence, as measured using one of the sequence comparison algorithms disclosed herein or by visual inspection.

The term substantially identical in regards to a nucleotide or protein sequence means that a particular sequence varies from the sequence of a naturally occurring sequence by one or more deletions, substitutions, or additions, the net effect of which is to retain biological function of an ENOD93 nucleic acid or protein.

For comparison of two or more sequences, typically one sequence acts as a reference sequence to which one or more test sequences are compared. When using a sequence comparison algorithm, test and reference sequences are entered into a computer program, subsequence coordinates are designated if necessary, and sequence algorithm program parameters are selected. The sequence comparison algorithm then calculates the percent sequence identity for the designated test sequence(s) relative to the reference sequence, based on the selected program parameters.

Optimal alignment of sequences for comparison may be conducted, for example, by the local homology algorithm of Smith & Waterman, Adv. Appl. Math, 1981, 2:482-489, by the homology alignment algorithm of Needleman & Wunsch, J. Mol. Biol., 1970, 48:443-453, by the search for similarity method of Pearson & Lipman, Proc. Natl. Acad. Sci. USA, 1988, 85:2444-2448, by computerized implementations of these algorithms (GAP, BESTFIT, FASTA, and TFASTA in the Wisconsin Genetics Software Package, Genetics Computer Group, Madison, Wisconsin), or by visual inspection. *See generally,* Ausubel (ed.), Short Protocols in Molecular Biology, 3rd ed., 1995, Wiley, New York.

A preferred algorithm for determining percent sequence identity and sequence similarity is the BLAST algorithm, which is described in Altschul et al., J. Mol. Biol., 1990, 215:403-410. Software for performing BLAST analyses is publicly available through the National Center for Biotechnology Information (internet address ncbi.nlm.nih.gov/). The BLAST algorithm parameters determine the sensitivity and speed of the alignment. For comparison of two nucleotide sequences, the BLASTn default parameters are set at W=11 (wordlength) and E=10 (expectation), and also include use of a low-complexity filter to mask residues of the query sequence having low compositional complexity. For comparison of two amino acid sequences, the BLASTp program default parameters are set at W=3 (wordlength), E=10 (expectation), use of the BLOSUM62 scoring matrix, gap costs of existence=11 and extension=1, and use of a low-complexity filter to mask residues of the query sequence having low compositional complexity.

### II. System for Recombinant Expression of an OsENOD93 Protein

The present disclosure further provides a system for expression of a recombinant OsENOD93 protein. Such a system may be used for subsequent purification and/or characterization of an OsENOD93 protein. A system for recombinant expression of an OsENOD93 protein may also be used for identification of activators, or targets of an OsENOD93 protein, as described further herein below. An expression system refers to a host cell comprising a heterologous nucleic acid and the protein encoded by the heterologous nucleic acid. For example, a heterologous expression system may comprise a host cell transfected with a construct comprising an *OsENOD93* nucleic acid encoding an OsENOD93 protein operably linked to a promoter, or a cell line produced by introduction of OsENOD93 nucleic acids into a host cell genome. The expression system may further comprise one or more additional heterologous nucleic acids relevant to OsENOD93 function, such as targets of OsENOD93 activity. These additional nucleic acids may be expressed as a single construct or multiple constructs.

Isolated proteins and recombinantly produced proteins may be purified and characterized using a variety of standard techniques that are known to the skilled artisan. *See e.g.,* Schröder et al., The Peptides, 1965, Academic Press, New York; Bodanszky, Principles of Peptide Synthesis, 2nd rev. ed. 1993, Springer-Verlag, Berlin/ New York; Ausubel (ed.), Short Protocols in Molecular Biology, 3rd ed., 1995, Wiley, New York.

### II.A. Expression Constructs

A construct for expression of an OsENOD93 protein may include a vector sequence and an *OsENOD93* nucleotide sequence, wherein the *OsENOD93* nucleotide sequence is operably linked to a promoter sequence. A construct for recombinant OsENOD93 expression may also comprise transcription termination signals and sequences required for proper translation of the nucleotide sequence. Preparation of an expression construct, including addition of translation and termination signal sequences, is known to one skilled in the art.

The promoter may be any polynucleotide sequence which shows transcriptional activity in the chosen plant cells, plant parts, or plants. The promoter may be native or analogous, or foreign or heterologous, to the plant host and/or to the DNA sequence of the invention. Where the promoter is native or endogenous to the plant host, it is intended that the promoter is found in the native plant into which the promoter is introduced. Where the promoter is foreign or heterologous to the DNA sequence of the invention, the promoter is not the native or naturally occurring promoter for the operably linked DNA sequence of the invention. The promoter may be inducible or constitutive. It may be naturally-occurring, may be composed of portions of various naturally-occurring promoters, or may be partially or totally synthetic. Guidance for the design of promoters is provided by studies of promoter structure, such as that of Harley et al., Nucleic Acids Res., 1987, 15:2343-61. Also, the location of the promoter relative to the transcription start may be optimized. *See e.g.,* Roberts et al., Proc. Natl. Acad. Sci. USA, 1979, 76:760-4. Many suitable promoters for use in plants are well known in the art.

For example, suitable constitutive promoters for use in plants include the promoters from plant viruses, such as the peanut chlorotic streak caulimovirus (PC1SV) promoter (U.S. Patent No. 5,850,019); the 35S promoter from cauliflower mosaic virus (CaMV) (Odell et al., Nature, 1985, 313:810-812); promoters of *Chlorella* virus methyltransferase genes (U.S. Patent No. 5,563,328) and the full-length transcript promoter from figwort mosaic virus (FMV) (U.S. Patent No. 5,378,619); the promoters from such genes as rice actin (McElroy et al. (1990) Plant Cell 2:163-171); ubiquitin (Christensen et al., Plant Mol. Biol., 1989, 12:619-632 and Christensen et al., Plant Mol. Biol., 1992, 18:675-689); pEMU (Last et al., Theor. Appl. Genet., 1991, 81:581-588); MAS (Velten et al., EMBO J., 1984, 3:2723-2730); maize H3 histone (Lepetit et al., Mol. Gen. Genet., 1992, 231:276-285 and Atanassova et al., Plant J., 1992, 2(3):291-300); *Brassica napus* ALS3 (PCT International Publication No. WO 97/41228); and promoters of various *Agrobacterium* genes (*see* U.S. Patent Nos. 4,771,002; 5,102,796; 5,182,200; and 5,428,147).

Suitable inducible promoters for use in plants include the promoter from the ACE1 system which responds to copper (Mett et al., Proc. Natl. Acad. Sci. USA, 1993, 90:4567-4571); the promoter of the maize In2 gene which responds to benzenesulfonamide herbicide safeners (Hershey et al., Mol. Gen. Genetics, 1991, 227:229-237 and Gatz et al., Mol. Gen. Genetics, 1994, 243:32-38); and the promoter of the Tet repressor from Tn10 (Gatz et al., Mol. Gen. Genet., 1991, 227:229-237). Another inducible promoter for use in plants is one that responds to an inducing agent to which plants do not normally respond. An exemplary inducible promoter of this type is the inducible promoter from a steroid hormone gene, the transcriptional activity of which is induced by a glucocorticosteroid hormone (Schena et al., Proc. Natl. Acad. Sci. USA, 1991, 88:10421) or the recent application of a chimeric transcription activator, XVE, for use in an estrogen receptor-based inducible plant expression system activated by estradiol (Zuo et al., Plant J., 2000, 24:265-273). Other inducible promoters for use in plants are described in EP 332104, PCT International Publication Nos. WO 93/21334 and WO 97/06269. Promoters composed of portions of other promoters and partially or totally synthetic promoters can also be used. *See e.g.,* Ni et al., Plant J., 1995, 7:661-676 and PCT International Publication No. WO 95/14098 describing such promoters for use in plants.

The promoter may include, or be modified to include, one or more enhancer elements to thereby provide for higher levels of transcription. Suitable enhancer elements for use in plants include the PC1SV enhancer element (U.S. Patent No. 5,850,019), the CaMV 35S enhancer element (U.S. Patent Nos. 5,106,739 and 5,164,316) and the FMV enhancer element (Maiti et al., Transgenic Res., 1997, 6:143-156). *See also* PCT International Publication No. WO 96/23898.

Such constructs can contain a 'signal sequence' or 'leader sequence' to facilitate cotranslational or post-translational transport of the peptide of interest to certain intracellular structures such as the chloroplast (or other plastid), endoplasmic reticulum, or Golgi apparatus, or to be secreted. For example, the construct can be engineered to contain a signal peptide to facilitate transfer of the peptide to the endoplasmic reticulum. A signal sequence is known or suspected to result in cotranslational or post-translational peptide transport across the cell membrane. In eukaryotes, this typically involves secretion into the Golgi apparatus, with some resulting glycosylation. A leader sequence refers to any sequence that, when translated, results in an amino acid sequence sufficient to trigger co-translational transport of the peptide chain to a sub-cellular organelle. Thus, this includes leader sequences targeting transport and/or glycosylation by passage into the endoplasmic reticulum, passage to vacuoles, plastids including chloroplasts, mitochondria, and the like. Plant expression cassettes may also contain an intron, such that mRNA processing of the intron is required for expression.

Such constructs can also contain 5' and 3' untranslated regions. A 3' untranslated region is a polynucleotide located downstream of a coding sequence. Polyadenylation signal sequences and other sequences encoding regulatory signals capable of affecting the addition of polyadenylic acid tracts to the 3' end of the mRNA precursor are 3' untranslated regions. A 5' untranslated region is a polynucleotide located upstream of a coding sequence.

The termination region may be native with the transcriptional initiation region, may be native with the sequence of the present invention, or may be derived from another source. Convenient termination regions are available from the Ti-plasmid of *A. tumefaciens,* such as the octopine synthase and nopaline synthase termination regions. *See also* Guerineau et al., Mol. Gen. Genet., 1991, 262:141-144; Proudfoot, Cell, 1991, 64:671-674; Sanfacon et al., Genes Dev. 1991, 5:141-149; Mogen et al., Plant Cell, 1990, 2:1261-1272; Munroe et al., Genes, 1990, 91:151-158; Ballas et al., Nucleic Acids Res., 1989, 17:7891-7903; and Joshi et al., Nucleic Acid Res., 1987, 15:9627-9639.

Where appropriate, the vector and *OsENOD93* sequences may be optimized for increased expression in the transformed host cell. That is, the sequences can be synthesized using host cell-preferred codons for improved expression, or may be synthesized using codons at a host-preferred codon usage frequency. Generally, the GC content of the polynucleotide will be increased. *See e.g.,* Campbell et al., Plant Physiol., 1990, 92:1-11 for a discussion of host-preferred codon usage. Methods are known in the art for synthesizing host-preferred polynucleotides. *See e.g.,* U.S. Patent Nos. 6,320,100; 6,075,185; 5,380,831; and 5,436,391, U.S. Published Application Nos. 20040005600 and 20010003849, and Murray et al., Nucleic Acids Res., 1989, 17:477-498.

For example, polynucleotides of interest can be targeted to the chloroplast for expression. In this manner, where the polynucleotide of interest is not directly inserted into the chloroplast, the expression cassette may additionally contain a polynucleotide encoding a transit peptide to direct the nucleotide of interest to the chloroplasts. Such transit peptides are known in the art. *See e.g.,* Von Heijne et al., Plant Mol. Biol. Rep., 1991, 9:104-126; Clark et al. J. Biol. Chem., 1989, 264:17544-17550; Della-Cioppa et al., Plant Physiol., 1987, 84:965-968; Romer et al., Biochem. Biophys. Res. Commun., 1993, 196:1414-1421; and Shah et al., Science, 1986, 233:478-481. The polynucleotides of interest to be targeted to the chloroplast may be optimized for expression in the chloroplast to account for differences in codon usage between the plant nucleus and this organelle. In this manner, the polynucleotides of interest may be synthesized using chloroplast-prefered codons. *See e.g.*, U.S Patent No. 5,380,831.

A plant expression cassette (*i.e*., an *OsENOD93* open reading frame operably linked to a promoter) can be inserted into a plant transformation vector, which allows for the transformation of DNA into a cell. Such a molecule may consist of one or more expression cassettes, and may be organized into more than one vector DNA molecule. For example, binary vectors are plant transformation vectors that utilize two non-contiguous DNA vectors to encode all requisite cis-and trans-acting functions for transformation of plant cells (Hellens et al., Trends in Plant Science, 2000, 5:446-451).

A plant transformation vector comprises one or more DNA vectors for achieving plant transformation. For example, it is a common practice in the art to utilize plant transformation vectors that comprise more than one contiguous DNA segment. These vectors are often referred to in the art as binary vectors. Binary vectors as well as vectors with helper plasmids are most often used for *Agrobacterium*-mediated transformation, where the size and complexity of DNA segments needed to achieve efficient transformation is quite large, and it is advantageous to separate functions onto separate DNA molecules. Binary vectors typically contain a plasmid vector that contains the cis-acting sequences required for T-DNA transfer (such as left border and right border), a selectable marker that is engineered to be capable of expression in a plant cell, and a polynucleotide of interest *(i.e.,* a polynucleotide engineered to be capable of expression in a plant cell for which generation of transgenic plants is desired). Also present on this plasmid vector are sequences required for bacterial replication. The cis-acting sequences are arranged in a fashion to allow efficient transfer into plant cells and expression therein. For example, the selectable marker sequence and the sequence of interest are located between the left and right borders. Often a second plasmid vector contains the trans-acting factors that mediate T-DNA transfer from *Agrobacterium* to plant cells. This plasmid often contains the virulence functions (*Vir* genes) that allow infection of plant cells by *Agrobacterium,* and transfer of DNA by cleavage at border sequences and vir-mediated DNA transfer, as in understood in the art (Hellens et al., 2000). Several types of *Agrobacterium* strains (e.g., LBA4404, GV3101, EHA101, EHA105, *etc.*) can be used for plant transformation. The second plasmid vector is not necessary for introduction of polynucleotides into plants by other methods such as microprojection, microinjection, electroporation, polyethylene glycol, *etc.*

### II.B. Host Cells

Host cells are cells into which a heterologous nucleic acid molecule of the invention may be introduced. Representative eukaryotic host cells include yeast and plant cells, as well as prokaryotic hosts such as *E.coli* and *Bacillus subtilis.* Preferred host cells for functional assays substantially or completely lack endogenous expression of an OsENOD93 protein.

A host cell strain may be chosen which effects the expression of the recombinant sequence, or modifies and processes the gene product in a specific manner. For example, different host cells have characteristic and specific mechanisms for the translational and post-translational processing and modification (e.g., glycosylation, phosphorylation of proteins). Appropriate cell lines or host cells may be chosen to ensure the desired modification and processing of the foreign protein expressed. For example, expression in a bacterial system may be used to produce a non-glycosylated core protein product, and expression in yeast will produce a glycosylated product.

The present invention further encompasses recombinant expression of an OsENOD93 protein in a stable cell line. Methods for generating a stable cell line following transformation of a heterologous construct into a host cell are known in the art. *See e.g.,* Joyner, Gene Targeting: A Practical Approach, 1993, Oxford University Press, Oxford/New York. Thus, transformed cells, tissues, and plants are understood to encompass not only the end product of a transformation process, but also transgenic progeny or propagated forms thereof.

### III. OsENOD93 Overexpressing Plants

The present invention also provides *OsENOD93* overexpressing plants comprising an overexpressed OsENOD93 nucleic acid or protein. The present invention also provides the generation of plants with conditional or inducible expression of OsENOD93.

*OsENOD93* overexpressing plants may be prepared in monocot or dicot plants, for example, in corn (maize), sorghum, wheat, sunflower, tomato, crucifers, peppers, potato, cotton, rice, soybean, sugarbeet, sugarcane, tobacco, barley, and oilseed rape, *Brassica sp.,* alfalfa, rye, millet, safflower, peanuts, sweet potato, cassava, coffee, coconut, pineapple, citrus trees, cocoa, tea, banana, avocado, fig, guava, mango, olive, papaya, cashew, macadamia, almond, oats, vegetables, ornamentals, and conifers. Representative vegatables include tomatoes, lettuce, green beans, lima beans, peas, yams, onion, and members of the genus *Curcumis* such as cucumber, cantaloupe, and musk melon. Ornamentals include, but are not limited to, azalea, hydrangea, hibiscus, roses, tulips, daffodils, petunias, carnation, poinsettia, and chrysanthemum. As used herein, a plant refers to a whole plant, a plant organ (*e.g.,* leaves, stems, roots, *etc*.), a seed, a plant cell, a propagule, an embryo, and progeny of the same. Plant cells can be differentiated or undifferentiated (e.g., callus, suspension culture cells, protoplasts, leaf cells, root cells, phloem cells, pollen).

The *OsENOD93* overexpressing plants may be further modified at a locus other than *OsENOD93* to confer enhanced nitrogen use efficiency or other trait of interest. Representative desired traits include improved crop yield; increased seed yield; increased amino acid content; increased nitrate content; increased tolerance to stress; insect resistance; tolerance to broad-spectrum herbicides; resistance to diseases caused by viruses, bacteria, fungi, and worms; and enhancement of mechanisms for protection from environmental stresses such as heat, cold, drought, and high salt concentration. Additional desired traits include output traits that benefit consumers, for example, nutritionally enhanced foods that contain more starch or protein, more vitamins, more anti-oxidants, and/or fewer trans-fatty acids; foods with improved taste, increased shelf-life, and better ripening characteristics; trees that make it possible to produce paper with less environmental damage; nicotine-free tobacco; ornamental flowers with new colors, fragrances, and increased longevity; *etc.* Still further, desirable traits that may be used in accordance with the disclosure include gene products produced in plants as a means for manufacturing, for example, therapeutic proteins for disease treatment and vaccination; textile fibers; biodegradable plastics; oils for use in paints, detergents, and lubricants; *etc.* For genetic modifications that confer traits associated with altered gene expression, nitrate content, amino acid content, nitrate uptake, lateral root growth, or plant biomass, the combination of an *OsENOD93* overexpressing plant and a second genetic modification can produce a synergistic effect, *i.e.*, a change in gene expression, nitrate content, amino acid content, nitrate uptake, lateral root growth, or plant biomass that is greater than the change elicited by either genetic modification alone.

For preparation of an *OsENOD93* overexpressing plant, introduction of a polynucleotide into plant cells is accomplished by one of several techniques known in the art, including but not limited to electroporation or chemical transformation (*See e.g.,* Ausubel, ed. (1994) Current Protocols in Molecular Biology, John Wiley and Sons, Inc., Indianapolis, Indiana. Markers conferring resistance to toxic substances are useful in identifying transformed cells (having taken up and expressed the test polynucleotide sequence) from non-transformed cells (those not containing or not expressing the test polynucleotide sequence). In one aspect of the invention, genes are useful as a marker to assess introduction of DNA into plant cells. Transgenic plants, transformed plants, or stably transformed plants, or cells, tissues or seed of any of the foregoing, refer to plants that have incorporated or integrated exogenous polynucleotides into the plant cell. Stable transformation refers to introduction of a polynucleotide construct into a plant such that it integrates into the genome of the plant and is capable of being inherited by progeny thereof.

In general, plant transformation methods involve transferring heterologous DNA into target plant cells (e.g., immature or mature embryos, suspension cultures, undifferentiated callus, protoplasts, *etc.),* followed by applying a maximum threshold level of appropriate selection (depending on the selectable marker gene) to recover the transformed plant cells from a group of untransformed cell mass. Explants are typically transferred to a fresh supply of the same medium and cultured routinely. Subsequently, the transformed cells are differentiated into shoots after placing on regeneration medium supplemented with a maximum threshold level of selecting agent (i.e., temperature and/or herbicide). The shoots are then transferred to a selective rooting medium for recovering rooted shoot or plantlet. The transgenic plantlet then grow into mature plant and produce fertile seeds (e.g., Hiei et al., Plant J., 1994, 6:271-282; Ishida et al., Nat. Biotechnol., 1996, 14:745-750). A general description of the techniques and methods for generating transgenic plants are found in Ayres et al., CRC Crit. Rev. Plant Sci., 1994. 13:219-239, and Bommineni et al., Maydica, 1997, 42:107-120. Since the transformed material contains many cells, both transformed and non-transformed cells are present in any piece of subjected target callus or tissue or group of cells. The ability to kill non-transformed cells and allow transformed cells to proliferate results in transformed plant cultures. Often, the ability to remove non-transformed cells is a limitation to rapid recovery of transformed plant cells and successful generation of transgenic plants. Then molecular and biochemical methods can be used for confirming the presence of the integrated nucleotide(s) of interest in the genome of transgenic plant.

Generation of transgenic plants may be performed by one of several methods, including but not limited to introduction of heterologous DNA by *Agrobacterium* into plant cells (*Agrobacterium*-mediated transformation), bombardment of plant cells with heterologous foreign DNA adhered to particles, and various other non-particle direct-mediated methods (e.g., Hiei et al., Plant J., 1994, 6:271-282; Ishida et al., Nat. Biotechnol., 1996, 14:745-750; Ayres et al., CRC Crit. Rev. Plant Sci., 1994, 13:219-239; Bommineni et al., Maydica, 1997, 42:107-120) to transfer DNA.

There are three common methods to transform plant cells with *Agrobacterium.* The first method is co-cultivation of *Agrobacterium* with cultured isolated protoplasts. This method requires an established culture system that allows culturing protoplasts and plant regeneration from cultured protoplasts. The second method is transformation of cells or tissues with *Agrobacterium.* This method requires (a) that the plant cells or tissues can be transformed by *Agrobacterium* and (b) that the transformed cells or tissues can be induced to regenerate into whole plants. The third method is transformation of seeds, apices or meristems with *Agrobacterium.* This method requires micropropagation.

The efficiency of transformation by *Agrobacterium* may be enhanced by using a number of methods known in the art. For example, the inclusion of a natural wound response molecule such as acetosyringone (AS) to the *Agrobacterium* culture has been shown to enhance transformation efficiency with *Agrobacterium tumefaciens* (Shahla et al., Plant Molec. Biol, 1987, 8:291-298). Alternatively, transformation efficiency may be enhanced by wounding the target tissue to be transformed. Wounding of plant tissue may be achieved, for example, by punching, maceration, bombardment with microprojectiles, *etc. See e.g.,* Bidney et al., Plant Molec. Biol., 1992, 18:301-313.

In another aspect of the invention, the plant cells are transfected with vectors via particle bombardment (*i.e.*, with a gene gun). Particle mediated gene transfer methods are known in the art, are commercially available, and include, but are not limited to, the gas driven gene delivery instrument described in U.S. Patent No. 5,584,807. This method involves coating the polynucleotide sequence of interest onto heavy metal particles, and accelerating the coated particles under the pressure of compressed gas for delivery to the target tissue.

Other particle bombardment methods are also available for the introduction of heterologous polynucleotide sequences into plant cells. Generally, these methods involve depositing the polynucleotide sequence of interest upon the surface of small, dense particles of a material such as gold, platinum, or tungsten. The coated particles are themselves then coated onto either a rigid surface, such as a metal plate, or onto a carrier sheet made of a fragile material such as mylar. The coated sheet is then accelerated toward the target biological tissue. The use of the flat sheet generates a uniform spread of accelerated particles that maximizes the number of cells receiving particles under uniform conditions, resulting in the introduction of the polynucleotide sample into the target tissue.

Specific initiation signals may also be used to achieve more efficient translation of sequences encoding the polypeptide of interest. Such signals include the ATG initiation codon and adjacent sequences. In cases where sequences encoding the polypeptide of interest, its initiation codon, and upstream sequences are inserted into the appropriate expression vector, no additional transcriptional or translational control signals may be needed. However, in cases where only coding sequence, or a portion thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers that are appropriate for the particular cell system that is used, such as those described in the literature (Scharf et al., Results Probl. Cell Differ., 1994, 20:125).

In another aspect of the present invention, at least one genomic copy corresponding to a nucleotide sequence of the present invention is modified in the genome of the plant by homologous recombination as further illustrated in Paszkowski et al., EMBO Journal 1988, 7:4021-26. This technique uses the property of homologous sequences to recognize each other and to exchange nucleotide sequences between each by a process known in the art as homologous recombination. Homologous recombination can occur between the chromosomal copy of a nucleotide sequence in a cell and an incoming copy of the nucleotide sequence introduced in the cell by transformation. Specific modifications are thus accurately introduced in the chromosomal copy of the nucleotide sequence. In one aspect, the regulatory elements of the nucleotide sequences of the present invention are modified. Such regulatory elements are easily obtainable by screening a genomic library using the nucleotide sequences of the present invention, or a portion thereof, as a probe. The existing regulatory elements are replaced by different regulatory elements, thus altering expression of the nucleotide sequence.

The cells that have been transformed may be grown into plants in accordance with conventional ways. *See e.g.,* McCormick et al., Plant Cell Rep., 1986, 5:81-84. These plants may then be grown, and either pollinated with the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as transgenic seed) having a polynucleotide of the disclosure, for example, an expression cassette of the disclosure, stably incorporated into their genome.

Transgenic plants of the invention can be homozygous for the added polynucleotides; i.e., a transgenic plant that contains two added sequences, one sequence at the same locus on each chromosome of a chromosome pair. A homozygous transgenic plant can be obtained by sexually mating (selfing) an independent segregant transgenic plant that contains the added sequences according to the invention, germinating some of the seed produced and analyzing the resulting plants produced for enhanced enzyme activity (i.e., herbicide resistance) and/or increased plant yield relative to a control (native, non-transgenic) or an independent segregant transgenic plant.

It is to be understood that two different transgenic plants can also be mated to produce offspring that contain two independently segregating added, exogenous polynucleotides. Selfing of appropriate progeny can produce plants that are homozygous for all added, exogenous polynucleotides that encode a polypeptide of the present disclosure. Back-crossing to a parental plant and outcrossing with a non-transgenic plant are also contemplated.

Following introduction of DNA into plant cells, the transformation or integration of the polynucleotide into the plant genome is confirmed by various methods such as analysis of polynucleotides, polypeptides and metabolites associated with the integrated sequence.

### IV. OsENOD93 Binding Partners and Activators

The present disclosure further discloses assays to identify OsENOD93 binding partners and OsENOD93 activators. OsENOD93 activators are agents that alter chemical and biological activities or properties of an OsENOD93 protein. Such chemical and biological activities and properties may include, but are not limited to, OsENOD93 nucleic acid expression levels and expression levels of nucleic acids subject to OsENOD93 regulation. Methods of identifying activators involve assaying an enhanced, level or quality of OsENOD93 function in the presence of one or more test agents. Representative OsENOD93 activators include small molecules as well as biological entities, as described herein below.

A control level or quality of OsENOD93 activity refers to a level or quality of wild type OsENOD93 activity, for example, when using a recombinant expression system comprising expression of SEQ ID NO:3. When evaluating the activating capacity of a test agent, a control level or quality of OsENOD93 activity comprises a level or quality of activity in the absence of the test agent.

Significantly changed activity of an OsENOD93 protein refers to a quantifiable change in a measurable quality that is larger than the margin of error inherent in the measurement technique. For example, significant inhibition refers to OsENOD93 activity that is reduced by about 2-fold or greater relative to a control measurement, or an about 5-fold or greater reduction, or an about 10-fold or greater reduction. A significant enhancement refers to OsENOD93 activity that is increased by about 2-fold or greater relative to a control measurement, or an about 5-fold or greater increase, or an about 10-fold or greater increase.

An assay of OsENOD93 function may comprise determining a level of *OsENOD93* gene expression; determining DNA binding activity of a recombinantly expressed OsENOD93 protein; determining an active conformation of an OsENOD93 protein; or determining activation of signaling events in response to binding of a OsENOD93 activator (e.g., enhanced expression of nitrate transporters, increased nitrate content, increased amino acid content, increased nitrate uptake, increased lateral root sprouting, and/or increased biomass). For example, a method of identifying an OsENOD93 activator comprises (a) providing a cell expressing an OsENOD93 protein; (b) contacting the cell with one or more test agents or a control agent; (c) assaying expression of a nucleic acid encoding the OsENOD93 protein; and (d) selecting a test agent that induces elevated expression of the nucleic acid when contacted with the test agent as compared to the control agent.

In accordance with the present disclosure there is also provided a rapid and high throughput screening method that relies on the methods described herein. This screening method comprises separately contacting an OsENOD93 protein with a plurality of test agents. In such a screening method the plurality of test agents may comprise more than about 10⁴ samples, or more than about 10⁵ samples, or more than about 10⁶ samples.

The *in vitro* and cellular assays of the disclosure may comprise soluble assays, or may further comprise a solid phase substrate for immobilizing one or more components of the assay. For example, an OsENOD93 protein, or a cell expressing an OsENOD93 protein, may be bound directly to a solid state component via a covalent or non-covalent linkage. Optionally, the binding may include a linker molecule or tag that mediates indirect binding of an OsENOD93 protein to a substrate.

### IV.A. Test Agents

A test agent refers to any agent that potentially interacts with a OsENOD93 nucleic acid or protein, including any synthetic, recombinant, or natural product. A test agent suspected to interact with a protein may be evaluated for such an interaction using the methods disclosed herein.

Representative test agents include but are not limited to peptides, proteins, nucleic acids, small molecules (*e.g*., organic and inorganic chemical compounds), antibodies or fragments thereof, nucleic acid-protein fusions, any other affinity agent, and combinations thereof. A test agent to be tested may be a purified molecule, a homogenous sample, or a mixture of molecules or compounds.

A small molecule refers to a compound, for example an organic compound, with a molecular weight of less than about 1,000 daltons, more preferably less than about 750 daltons, still more preferably less than about 600 daltons; and still more preferably less than about 500 daltons. A small molecule also preferably has a computed log octanol-water partition coefficient in the range of about -4 to about +14, more preferably in the range of about -2 to about +7.5.

Test agents may be obtained or prepared as a library or collection of molecules. A library may contain a few or a large number of different molecules, varying from about ten molecules to several billion molecules or more. A molecule may comprise a naturally occurring molecule, a recombinant molecule, or a synthetic molecule. A plurality of test agents in a library may be assayed simultaneously. Optionally, test agents derived from different libraries may be pooled for simultaneous evaluation.

Representative libraries include but are not limited to a peptide library (U.S. Patent Nos. 6,156,511, 6,107,059, 5,922,545, and 5,223,409), an oligomer library (U.S. Patent Nos. 5,650,489 and 5,858,670), an aptamer library (U.S. Patent Nos. 7,338,762; 7,329,742; 6,949,379; 6,180,348; and 5,756,291), a small molecule library (U.S. Patent Nos. 6,168,912 and 5,738,996), a library of antibodies or antibody fragments (U.S. Patent Nos. 6,174,708, 6,057,098, 5,922,254, 5,840,479, 5,780,225, 5,702,892, and 5,667988), a library of nucleic acid-protein fusions (U.S. Patent No. 6,214,553), and a library of any other affinity agent that may potentially bind to an OsENOD93 protein.

A library may comprise a random collection of molecules. Alternatively, a library may comprise a collection of molecules having a bias for a particular sequence, structure, or conformation, for example, as for inhibitory nucleic acids. *See e.g.,* U.S. Patent Nos. 5,264,563 and 5,824,483. Methods for preparing libraries containing diverse populations of various types of molecules are known in the art, for example as described in U.S. patents cited herein above. Numerous libraries are also commercially available.

### IV.B. Binding Assays

In another aspect of the disclosure, a method of identifying of an OsENOD93 activator comprises determining specific binding of a test agent to an OsENOD93 protein. For example, a method of identifying an OsENOD93 binding partner may comprise: (a) providing an OsENOD93 protein of SEQ ID NO:3; (b) contacting the OsENOD93 protein with one or more test agents under conditions sufficient for binding; (c) assaying binding of a test agent to the isolated OsENOD93 protein; and (d) selecting a test agent that demonstrates specific binding to the OsENOD93 protein. Specific binding may also encompass a quality or state of mutual action such that binding of a test agent to an OsENOD93 protein is inhibitory or inducing.

Specific binding refers to a binding reaction which is determinative of the presence of the protein in a heterogeneous population of proteins and other biological materials. The binding of a test agent to an OsENOD93 protein may be considered specific if the binding affinity is about 1x10⁴M⁻¹ to about 1x10⁶M⁻¹ or greater. Specific binding also refers to saturable binding. To demonstrate saturable binding of a test agent to an OsENOD93 protein, Scatchard analysis may be carried out as described, for example, by Mak et al., J. Biol. Chem., 1989, 264:21613-21618.

Several techniques may be used to detect interactions between an OsENOD93 protein and a test agent without employing a known competitive inhibitor. Representative methods include, but are not limited to, Fluorescence Correlation Spectroscopy, Surface-Enhanced Laser Desorption/Ionization Time-Of-flight Spectroscopy, and BIACORE® technology, each technique described herein below. These methods are amenable to automated, high-throughput screening.

Fluorescence Correlation Spectroscopy (FCS) measures the average diffusion rate of a fluorescent molecule within a small sample volume. The sample size may be as low as 10³ fluorescent molecules and the sample volume as low as the cytoplasm of a single bacterium. The diffusion rate is a function of the mass of the molecule and decreases as the mass increases. FCS may therefore be applied to protein-ligand interaction analysis by measuring the change in mass and therefore in diffusion rate of a molecule upon binding. In a typical experiment, the target to be analyzed (*e.g*., an OsENOD93 protein) is expressed as a recombinant protein with a sequence tag, such as a poly-histidine sequence, inserted at the N-terminus or C-terminus. The expression is mediated in a host cell, such as *E.coli,* yeast, *Xenopus* oocytes, or mammalian cells. The protein is purified using chromatographic methods. For example, the poly-histidine tag may be used to bind the expressed protein to a metal chelate column such as Ni2+ chelated on iminodiacetic acid agarose. The protein is then labeled with a fluorescent tag such as carboxytetramethylrhodamine or BODIPY™ reagent (available from Molecular Probes of Eugene, Oregon). The protein is then exposed in solution to the potential ligand, and its diffusion rate is determined by FCS using instrumentation available from Carl Zeiss, Inc. (Thomwood of New York, New York). Ligand binding is determined by changes in the diffusion rate of the protein.

Surface-Enhanced Laser Desorption/Ionization (SELDI) was developed by Hutchens & Yip, Rapid Commun. Mass Spectrom., 1993, 7:576-580. When coupled to a time-of-flight mass spectrometer (TOF), SELDI provides a technique to rapidly analyze molecules retained on a chip. It may be applied to ligand-protein interaction analysis by covalently binding the target protein, or portion thereof, on the chip and analyzing by mass spectrometry the small molecules that bind to this protein (Worrall et al., Anal Chem., 1998, 70(4):750-756). In a typical experiment, a target protein (*e.g.*, an OsENOD93 protein) is recombinantly expressed and purified. The target protein is bound to a SELDI chip either by utilizing a poly-histidine tag or by other interaction such as ion exchange or hydrophobic interaction. A chip thus prepared is then exposed to the potential ligand via, for example, a delivery system able to pipet the ligands in a sequential manner (autosampler). The chip is then washed in solutions of increasing stringency, for example a series of washes with buffer solutions containing an increasing ionic strength. After each wash, the bound material is analyzed by submitting the chip to SELDI-TOF. Ligands that specifically bind a target protein are identified by the stringency of the wash needed to elute them.

BIACORE® relies on changes in the refractive index at the surface layer upon binding of a ligand to a target protein (*e.g*., an OsENOD93 protein) immobilized on the layer. In this system, a collection of small ligands is injected sequentially in a 2-5 microliter cell, wherein the target protein is immobilized within the cell. Binding is detected by surface plasmon resonance (SPR) by recording laser light refracting from the surface. In general, the refractive index change for a given change of mass concentration at the surface layer is practically the same for all proteins and peptides, allowing a single method to be applicable for any protein. In a typical experiment, a target protein is recombinantly expressed, purified, and bound to a BIACORE® chip. Binding may be facilitated by utilizing a poly-histidine tag or by other interaction such as ion exchange or hydrophobic interaction. A chip thus prepared is then exposed to one or more potential ligands via the delivery system incorporated in the instruments sold by Biacore (Uppsala, Sweden) to pipet the ligands in a sequential manner (autosampler). The SPR signal on the chip is recorded and changes in the refractive index indicate an interaction between the immobilized target and the ligand. Analysis of the signal kinetics of on rate and off rate allows the discrimination between non-specific and specific interaction. *See also* Homola et al., Sensors and Actuators, 1999, 54:3-15 and references therein.

### IV.C. Conformational Assay

The present disclosure also provides methods of identifying OsENOD93 binding partners and activators that rely on a conformational change of an OsENOD93 protein when bound by or otherwise interacting with a test agent. For example, application of circular dichroism to solutions of macromolecules reveals the conformational states of these macromolecules. The technique may distinguish random coil, alpha helix, and beta chain conformational states.

To identify binding partners and activators of an OsENOD93 protein, circular dichroism analysis may be performed using a recombinantly expressed ENOD93 protein. An OsENOD93 protein is purified, for example by ion exchange and size exclusion chromatography, and mixed with a test agent. The mixture is subjected to circular dichroism. The conformation of an OsENOD93 protein in the presence of a test agent is compared to a conformation of an OsENOD93 protein in the absence of the test agent. A change in conformational state of an OsENOD93 protein in the presence of a test agent identifies an OsENOD93 binding partner or activator. Representative methods are described in U.S. Patent Nos. 5,776,859 and 5,780,242. Activity of the binding partner or activator may be assessed using functional assays, such assays include nitrate content, nitrate uptake, lateral root growth, seed yield, amino acid content or plant biomass, as described herein.

### IV.D. Functional Assays

In another aspect of the disclosure, a method of identifying an OsENOD93 activator employs a functional OsENOD93 protein, for example, as set forth in SEQ ID NO:3. Representative methods for determining OsENOD93 function include assaying a physiological change elicited by OsENOD93 activity, for example enhanced lateral root growth, increased nitrate uptake, increased nitrate content, increased seed yield, increased amino acid content and/or increased plant biomass *(See, e.g.,* Examples 1, 3-6, 10 and 12).

For example, a method of identifying an OsENOD93 activator may comprise (a) providing a cell expressing an OsENOD93 protein; (b) contacting the cell with one or more test agents or a control agent; (c) assaying expression of an OsENOD93 target gene; and (d) selecting a test agent that induces elevated expression of the OsENOD93 target gene, which gene is normally induced by OsENOD93 (*e.g*., nitrogen metabolism genes, carbon metabolism genes and photosynthate transporting genes), when contacted with the test agent as compared to the control agent. In accordance with the disclosed methods, cells expressing OsENOD93 may be provided in the form of a kit useful for performing an assay of OsENOD93 function. For example, a test kit for detecting an OsENOD93 activator may include cells transfected with DNA encoding a full-length OsENOD93 protein and a medium for growing the cells.

Assays of OsENOD93 activity that employ transiently transfected cells may include a marker that distinguishes transfected cells from non-transfected cells. A marker may be encoded by or otherwise associated with a construct for OsENOD93 expression, such that cells are simultaneously transfected with a nucleic acid molecule encoding OsENOD93 and the marker. Representative detectable molecules that are useful as markers include but are not limited to a heterologous nucleic acid, a protein encoded by a transfected construct (e.g., an enzyme or a fluorescent protein), a binding protein, and an antigen.

A method of identifying an OsENOD93 activator may also comprise (a) providing a plant expressing an OsENOD93 protein; (b) contacting the plant with one or more test agents or a control agent; (c) assaying (i) nitrate content; (ii) nitrate uptake; (iii) amino acid content; (iv) seed yield; or (v) biomass; and (d) selecting a test agent that induces (i) increased nitrate content, (ii) increased nitrate uptake, (iii) increased amino acid content, (iv) increased seed yield, or (v) increased biomass.

Assays employing cells expressing recombinant OsENOD93 or plants expressing OsENOD93 may additionally employ control cells or plants that are substantially devoid of native OsENOD93 and, optionally, proteins substantially similar to an OsENOD93 protein. When using transiently transfected cells, a control cell may comprise, for example, an untransfected host cell. When using a stable cell line expressing an OsENOD93 protein, a control cell may comprise, for example, a parent cell line used to derive the OsENOD93-expressing cell line. When using plants, a control plant may include an *OsENOD93* overexpressing plant. In this instance, an OsENOD93 activator elicits a phenotype similar to an *OsENOD93* overexpressing plant.

### IV.E. Rational Design

The knowledge of the structure of a native OsENOD93 protein provides an approach for rational design of OsENOD93 activators. In brief, the structure of an OsENOD93 protein may be determined by X-ray crystallography and/or by computational algorithms that generate three-dimensional representations. *See* Saqi et al., Bioinformatics, 1999, 15:521-522; Huang et al., Pac. Symp. Biocomput, 2000, 230-241; and PCT International Publication No. WO 99/26966. Alternatively, a working model of an ENOD93 protein structure may be derived by homology modeling (Maalouf et al., J. Biomol. Struct. Dyn., 1998, 15(5):841-851). Computer models may further predict binding of a protein structure to various substrate molecules that may be synthesized and tested using the assays described herein above. Additional compound design techniques are described in U.S. Patent Nos. 5,834,228 and 5,872,011.

An OsENOD93 protein is a soluble protein, which may be purified and concentrated for crystallization. The purified OsENOD93 protein may be crystallized under varying conditions of at least one of the following: pH, buffer type, buffer concentration, salt type, polymer type, polymer concentration, other precipitating ligands, and concentration of purified OsENOD93. Methods for generating a crystalline protein are known in the art and may be reasonably adapted for determination of an OsENOD93 protein as disclosed herein. *See e.g.,* Deisenhofer et al., J. Mol. Biol., 1984, 180:385-398; Weiss et al., FEBS Lett., 1990, 267:268-272; or the methods provided in a commercial kit, such as the CRYSTAL SCREEN™ kit (available from Hampton Research of Riverside, California, USA).

A crystallized OsENOD93 protein may be tested for functional activity and differently sized and shaped crystals are further tested for suitability in X-ray diffraction. Generally, larger crystals provide better crystallography than smaller crystals, and thicker crystals provide better crystallography than thinner crystals. Preferably, OsENOD93 crystals range in size from 0.1-1.5 mm. These crystals diffract X-rays to at least 10 Å resolution, such as 1.5-10.0 Å or any range of value therein, such as 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5 or 3, with 3.5 Å or less being preferred for the highest resolution.

### IV.F. OsENOD93 Antibodies

In another aspect of the disclosure, a method is provided for producing an antibody that specifically binds an OsENOD93 protein. According to the method, a full-length recombinant OsENOD93 protein is formulated so that it may be used as an effective immunogen, and used to immunize an animal so as to generate an immune response in the animal. The immune response is characterized by the production of antibodies that may be collected from the blood serum of the animal.

An antibody is an immunoglobulin protein, or antibody fragments that comprise an antigen binding site (*e.g*., Fab, modified Fab, Fab', F(ab')₂ or Fv fragments, or a protein having at least one immunoglobulin light chain variable region or at least one immunoglobulin heavy chain region). Antibodies of the disclosure include diabodies, tetrameric antibodies, single chain antibodies, tretravalent antibodies, multispecific antibodies (*e.g*., bispecific antibodies), and domain-specific antibodies that recognize a particular epitope. Cell lines that produce anti-OsENOD93 antibodies are also encompassed by the disclosure.

Specific binding of an antibody to an OsENOD93 protein refers to preferential binding to an OsENOD93 protein in a heterogeneous sample comprising multiple different antigens. Substantially lacking binding describes binding of an antibody to a control protein or sample, *i.e.,* a level of binding characterized as non-specific or background binding. The binding of an antibody to an antigen is specific if the binding affinity is at least about 10⁻⁷ M or higher, such as at least about 10⁻⁸ M or higher, including at least about 10⁻⁹ M or higher, at least about 10⁻¹¹ M or higher, or at least about 10⁻¹² M or higher.

OsENOD93 antibodies prepared as disclosed herein may be used in methods known in the art relating to the expression and activity of OsENOD93 proteins, *e.g*., for cloning of nucleic acids encoding an OsENOD93 protein, immunopurification of an OsENOD93 protein, and detecting an OsENOD93 protein in a plant sample, and measuring levels of an OsENOD93 protein in plant samples. To perform such methods, an antibody of the present disclosure may further comprise a detectable label, including but not limited to a radioactive label, a fluorescent label, an epitope label, and a label that may be detected *in vivo.* Methods for selection of a label suitable for a particular detection technique, and methods for conjugating to or otherwise associating a detectable label with an antibody are known to one skilled in the art.

### V. Overexpression of OsENOD93

The disclosed OsENOD93 binding partners and OsENOD93 activators are useful both in *vitro* and *in vivo* for applications generally related to assessing responses to nitrogen levels and for promoting nitrogen use efficiency. In particular, OsENOD93 activators may be used to induce transcription of OsENOD93, to increase nitrate content in plants, to increase nitrate uptake into plant roots, to increase seed yield in plants, to increase amino acid content in plants and to increase plant biomass.

The present disclosure provides that an effective amount of an OsENOD93 activator is administered to a plant, *i.e.,* an amount sufficient to elicit a desired biological response. For example, an effective amount of an OsENOD93 activator may comprise an amount sufficient to elicit elevated expression of *OsENOD93,* genes normally subject to OsENOD93 induction (*e.g.*, nitrogen metabolism genes, carbon metabolism genes and photosynthate transporting genes), increased nitrate content in plant roots, increased nitrate uptake in plant roots, increased lateral root sprouting, increased seed yield, increased amino acid content and increased biomass.

Plants that may benefit from OsENOD93 activation include, but are not limited to, corn (maize), sorghum, wheat, sunflower, tomato, crucifers, peppers, potato, cotton, rice, soybean, sugarbeet, sugarcane, tobacco, barley, and oilseed rape, Brassica sp., alfalfa, rye, millet, safflower, peanuts, sweet potato, cassava, coffee, coconut, pineapple, citrus trees, cocoa, tea, banana, avocado, fig, guava, mango, olive, papaya, cashew, macadamia, almond, oats, vegetables, ornamentals, and conifers. Representative vegetables include tomatoes, lettuce, green beans, lima beans, peas, yams, onions, and members of the genus *Curcumis* such as cucumber, cantaloupe, and musk melon. Representative ornamentals include, but are not limited to, azalea, hydrangea, hibiscus, roses, tulips, daffodils, petunias, carnation, poinsettia, and chrysanthemum. Any of the afore-mentioned plants may be wild type, inbred, or transgenic, *e.g*., plants strains and genetically modified plants as used in agricultural settings.

Plants treated with an OsENOD93 activator may be transgenic, *i.e.,* genetically modified at *OsENOD93,* or at a locus other than *OsENOD93* to confer enhanced nitrogen use efficiency or other trait of interest. Representative desired traits include improved crop yield; increased seed yield; increased amino acid content; increased nitrate content; increased tolerance to stress; insect resistance; tolerance to broad-spectrum herbicides; resistance to diseases caused by viruses, bacteria, fungi, and worms; and enhancement of mechanisms for protection from environmental stresses such as heat, cold, drought, and high salt concentration. Additional desired traits include output traits that benefit consumers, for example, nutritionally enhanced foods that contain more starch or protein, more vitamins, more anti-oxidants, and/or fewer trans-fatty acids; foods with improved taste, increased shelf-life, and better ripening characteristics; trees that make it possible to produce paper with less environmental damage; nicotine-free tobacco; ornamental flowers with new colors, fragrances, and increased longevity; *etc.* Still further, desirable traits that may be used in accordance with the disclosure include gene products produced in plants as a means for manufacturing, for example, therapeutic proteins for disease treatment and vaccination; textile fibers; biodegradable plastics; oils for use in paints, detergents, and lubricants; *etc.* For genetic modifications that confer traits associated with increased altered gene expression, nitrate content, nitrate uptake, lateral root growth, seed yield, amino acid content or plant biomass, the combination of treatment with an OsENOD93 activator and a genetic modification can produce a synergistic effect, *i.e.,* a change in gene expression, nitrate content, nitrate uptake, lateral root growth, seed yield, amino acid content or plant biomass that is greater than the change elicited by either genetic modification alone.

### EXAMPLES

The following examples have been included to illustrate modes of the invention. Certain aspects of the following examples are described in terms of techniques and procedures found or contemplated by the present co-inventors to work well in the practice of the invention. In light of the present disclosure and the general level of skill in the art, those of skill will appreciate that the following examples are intended to be exemplary only and that numerous changes, modifications, and alterations may be employed without departing from the scope of the invention.

### Example 1. Changes In Biomass In Plants Exposed To Different Nitrogen Levels

Wild-type *Oryza sativa* Japonica cv. Donjin plants were grown in a mixture of peat moss and vermiculite (1:4) (SunGro Horticulture Canada Ltd. BC, Canada) with the addition of a nutrient solution with different amount of nitrate once a week till harvest. The nutrient solution contained 4 mM MgSO₄, 5 mM KCl, 5 mM CaCl₂, 1 mM KH₂PO₄, 0.1 mM Fe-EDTA, 0.5 mM MES (pH6.0), 9 µ M MnSO₄, 0.7 µM Zn SO₄, 0.3 µM CuSO₄, 46 µM NaB₄O₇ and 0.2 µM (NH₄)₆Mo₇O₂. 10 mM nitrate was used as the high nitrogen condition, 5 mM nitrate as medium nitrogen, and 1 mM nitrate as low nitrogen. Plants were grown in a growth room with 16 hr light (∼400 µmolm⁻²s⁻¹) at 28-30°C and 8 hr dark at 22-24°C for four weeks. Shoots and roots were harvested separately and assessed for differences in biomass as a marker of growth.

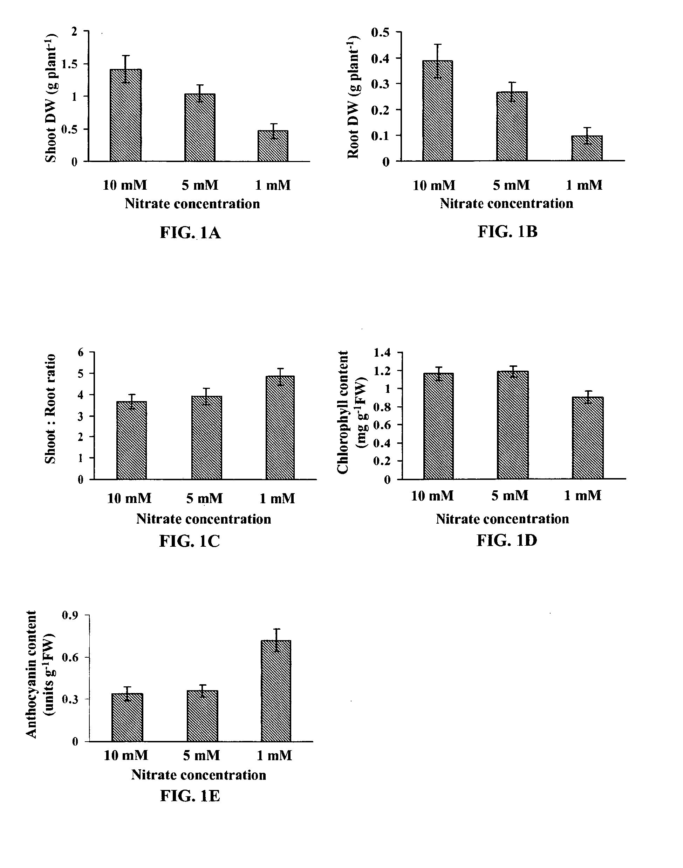

Under medium nitrogen concentration (5mM nitrate) plant growth measured by shoot biomass was reduced to approximately 70% of that at high nitrogen concentration (10 mM nitrate) (73% of dry weight), and under low nitrogen concentration (1 mM nitrate) plant growth measured by shoot biomass was further reduced to approximately 30% of that at high nitrogen concentration (10 mM nitrate) (33% of dry weight) (Figure 1A). Reduction of root biomass under the two limiting nitrogen concentrations was slightly more than shoot, which was 69% and 25% of dry weight respectively (Figure 1B), resulting in an increased shoot/root ratio with an increased nitrogen stress (Figure 1C).

### Example 2. Response To Nitrogen Deficiency

Wild-type *Oryza sativa* Japonica cv. Donjin plants were grown as described in Example 1. Leaf yellowing and the presence of the purple flavonoid anthocyanin are some of the typical responses plants have when N deficiency occurs (Diaz U, et al., Plant and Cell Physiology, 2006, 47: 74-83). Relative anthocyanin content was analyzed based on the method described by Neff and Chory (Neff MM & Chory J, Plant Physiol, 1998, 118: 27-35). Total chlorophyll was measured either using the Minolta SPAD 502DL chlorophyll meter (Tokyo, Japan), or extracted by ethanol and measured by spectrophotometer according to Kirk (Kirk, JTO, Planta, Berl., 1968, 78:200). Both chlorophyll and anthocyanin levels in the leaves were similar under high and medium nitrogen conditions. Under a low nitrogen condition, the plants were not obvious yellow or purple although the reduction of chlorophyll and the increase of anthocyanin were significant when compared to the high and medium nitrogen conditions (Figures 1D and 1E).

### Example 3. Changes In Free Nitrate Content In Plants Exposed To Different Nitrogen Levels

Wild-type *Oryza sativa* Japonica cv. Donjin plants were grown as described in Example 1. Frozen shoot and root tissue was used to determine differences in nitrate content under the different nitrogen conditions. Nitrate content was analyzed by colorimetric assay according to Cataldo DA, et al., Commun. Soil Sci. Plant Anal 1975, 6: 71-80.

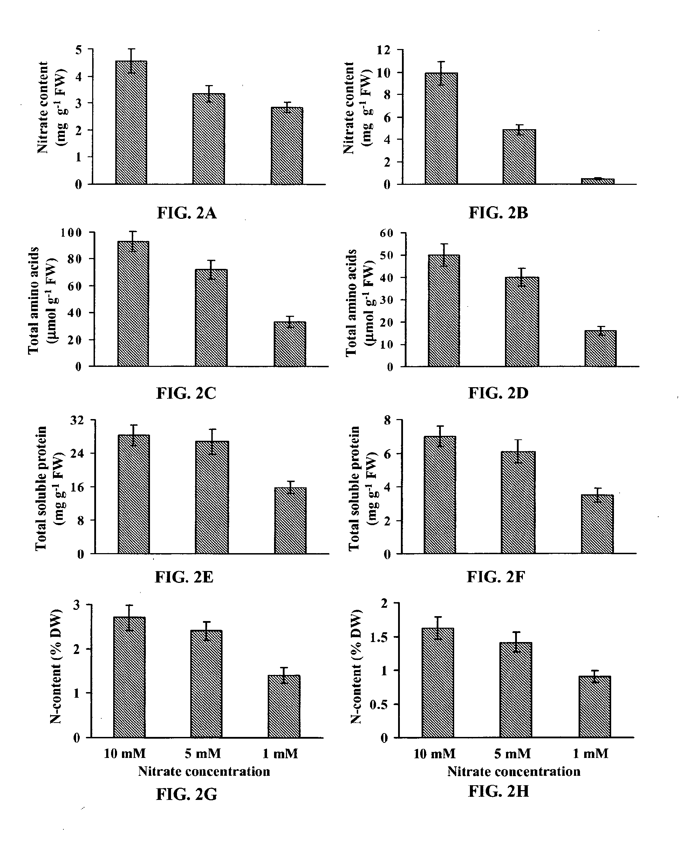

Free nitrate content in the shoots under medium nitrogen concentration (5mM nitrate) was reduced to 70% of that at high nitrogen concentration (10 mM nitrate) (3.35 mg/gFW vs. 4.55 mg/gFW), and not much further reduction under low nitrogen concentration (1mM nitrate) (2.85 mg/gFW vs. 4.55 mg/gFW) (Figure 2A). Free nitrate content in the roots was reduced by about half (4.8 mg/gFW vs. 9.9 mg/gFW) under medium nitrogen concentration (5mM nitrate) when compared to high nitrogen concentration (10 mM nitrate), and an almost 20-fold drop occurred under low nitrogen concentration (1mM nitrate) (0.45 mg/gFW vs. 9.9 mg/gFW) as compared to high nitrogen concentration (10 mM nitrate) (Figure 2B).

### Example 4. Changes In Amino Acid Content In Plants Exposed To Different Nitrogen Levels

Wild-type *Oryza sativa* Japonica cv. Donjin plants were grown as described in Example 1. Frozen shoot and root tissue was used to determine differences in amino acid content under the different nitrogen conditions. Total amino acids were extracted successively with 80%, 50% and 0% ethanol in 10 mM HEPES-KOH (pH 7.4), supernatants were pooled and total amino acids were assayed according to Rosen H, Arch. Biochem. Biophys. 1957, 67: 10-15.

Total amino acids content in the shoots under medium nitrogen concentration (5mM nitrate) was reduced to 77% of that at high nitrogen concentration (10 mM nitrate) and under low nitrogen concentration (1mM nitrate) was reduced to 35% of that at high nitrogen concentration (10 mM nitrate) (Figure 2C). A similar reduction was observed in the root (Figure 2D).

### Example 5. Changes In Soluble Protein Content In Plants Exposed To Different Nitrogen Levels

Wild-type *Oryza sativa* Japonica cv. Donjin plants were grown as described in Example 1. Frozen shoot and root tissue was used to determine differences in soluble protein content under the different nitrogen conditions. Total soluble protein was extracted with buffer containing 100 mM HEPES-KOH, pH 7.5, and 0.1% Triton X-100 and assayed using the Bio-Rad protein assay kit (Bio-Rad Laboratories, Inc., California, USA).

Total soluble protein in the shoots under medium nitrogen concentration (5mM nitrate) was similar to the level observed in shoots from plants grown under high nitrogen concentration (10mM), but was significantly lower under low nitrogen concentration (1mM) (Figure 2E). A similar result was observed in the roots grown under the same conditions (Figure 2F).

### Example 6. Changes In Total Nitrogen Content In Plants Exposed To Different Nitrogen Levels

Wild-type *Oryza sativa* Japonica cv. Donjin plants were grown as described in Example 1. Frozen shoot and root tissue was used to determine differences in total nitrogen content under the different nitrogen conditions. Percentage of total nitrogen in the dried tissues was measured by the Micro-Dumas combustion analysis method using a Carlo Erba NA1500 C/N analyzer, (Carlo Erba Strumentazione, Milan, Italy).

Percentage of total nitrogen in the shoots under medium nitrogen concentration (5mM nitrate) was similar to the level observed in shoots from plants grown under high nitrogen concentration (10mM), but was significantly lower under low nitrogen concentration (1mM) (Figure 2G). A similar result was observed in the roots grown under the same conditions (Figure 2H).

### Example 7. Identification Of Differentially Expressed Genes In Plants Exposed To Different Nitrogen Levels

Wild-type *Oryza sativa* Japonica cv. Donjin plants were grown as described in Example 1. Shoots and roots of 4-week-old wild-type rice plants grown under high nitrogen concentration (10 mM nitrate), medium nitrogen concentration (5 mM nitrate) and low nitrogen concentration (1 mM nitrate) were harvested to compare baseline gene expression levels under different but stable nitrogen concentrations. Each nitrogen concentration had three biological replicates. Additionally, at 2 hrs before the harvest, some plants grown under low nitrogen concentration (1 mM nitrate) were transferred to high nitrogen concentration (10 mM nitrate) to assess gene expression changes in response to 2 hr nitrogen induction. Other plants grown under high nitrogen concentration (10 mM nitrate) were transferred to low nitrogen concentration (1 mM nitrate) to assess gene expression changes in response to 2 hr nitrogen reduction. All samples were taken in the middle of the day to minimize diurnal changes in carbon and nitrogen metabolism. Three biological repeats were collected for each time point.

Five µg of total RNA from each sample was used to synthesize double-stranded cDNAs. Labeled complementary RNA, synthesized from the cDNA was hybridized to a custom designed rice GENECHIP® whole genome array (Zhu T, Current Opinion in Plant Biology 2003, 6: 418-425). The hybridization signal of the arrays was acquired by the GENECHIP® scanner 3000 and quantified by MAS 5.0 (Affymetrix, California, USA). The probe set measurement was summarized as a value of weighted average of all probes in a set, subtracting the bottom 5% of average intensity of the entire array using a custom algorithm. The overall intensity of all probe sets of each array was further scaled to a target intensity of 100 to enable direct comparison.

Eight comparisons were made between the following samples (four each for shoots and roots): (1) high nitrogen concentration vs. medium nitrogen concentration (10 mM nitrate to 5 mM nitrate); (2) high nitrogen concentration vs. low nitrogen concentration (10 mM nitrate to 1 mM nitrate); (3) low nitrogen concentration (1 mM nitrate) vs. low to high nitrogen concentration (2 hr nitrogen induction); and (4) high nitrogen concentration (10 mM nitrate) vs. high to low nitrogen concentration (2 hour nitrogen reduction).

The data collected was analyzed using GeneSpring (Agilent, California, USA). The data was normalized using the default setting of the program, followed by gene filtering, which required that each gene must have either a 'P' or 'M' flag in the 3 replicate samples. This was followed by a second filtering step requiring that at least one of the three samples had a 'P' flag. This essentially guaranteed that every gene remaining in a group would be 'PMM', 'PPM', or 'PPP' among the three replicates. For pairwise group comparisons, genes with 2-fold change were identified first, and then ANOVA was used to identify significant genes (Welch t-test p-value cutoff at 0.05).

To confirm the results of the microarray analysis, the expression of 8 significant genes from different comparison groups was tested by quantitative real-time PCR. Total RNA was isolated from plant tissues using TRIZOL reagent (Invitrogen, California, USA). To eliminate any residual genomic DNA, total RNA was treated with RQ1 RNase-free DNase (Promega, Wisconsin, USA). The first strand cDNA was synthesized from total RNA by using the Reverse Transcription System kit (Promega). PRIMER EXPRESS® 2.0 software (Applied Biosystems, California, USA) was used to design the primers. Real-time PCR was performed essentially according to Kant S, et al., Plant Cell Environ., 2006, 29: 1220-1234. Relative quantification (RQ) values for each target gene were calculated by the 2^{-ΔΔCT} method (Livak KJ, Schmittgen TD, Methods, 2001, 25: 402-408) using *ACTIN2* as an internal reference gene for comparing data from different PCR runs or cDNA samples. To ensure the validity of the 2^{-ΔΔCT} method, twofold serial dilutions of cDNA from control plants of *Thellungiella* and *Arabidopsis* were used to create standard curves, and the amplification efficiencies of the target and reference genes shown to be approximately equal (Livak and Schmittgen, 2001). The results of relative expression level were very consistent with the microarray data. The baseline expression levels of all the genes remained similar between the samples grown under high and medium nitrogen concentrations, both for shoots and roots (Table 1). However, the transcriptional change between root and shoot under low nitrogen concentration was different, as 59 significant genes were identified in the root samples (27 up-regulated, and 32 down-regulated), but none in the shoot sample (Table 1).

**Table 1**

| Nitrogen Treatment | Shoot | | Root | |
|---|---|---|---|---|
| | Up | Down | Up | Down |
| High vs. medium | 0 | 0 | 0 | 0 |
| High vs. low | 0 | 0 | 27 | 32 |
| Low vs. induction | 0 | 0 | 273 | 22 |
| High vs. reduction | 0 | 0 | 339 | 62 |

### Example 8. Response In Plants Subjected To Transient Changes In Nitrogen Levels

Wild-type *Oryza sativa* Japonica cv. Donjin plants were grown as described in Example 1 and subjected to the transient changes in nitrogen concentration described in Example 7, *i.e*., a 2 hour nitrogen induction (low nitrogen concentration (1 mM nitrate) to high nitrogen concentration (10 mM nitrate)) and a 2 hour nitrogen reduction (high nitrogen concentration (10 mM nitrate) to low nitrogen concentration (1 mM nitrate)). Nitrate content was determined in roots and shoots as described in Example 3 and differential gene expression was determined as described in Example 7.

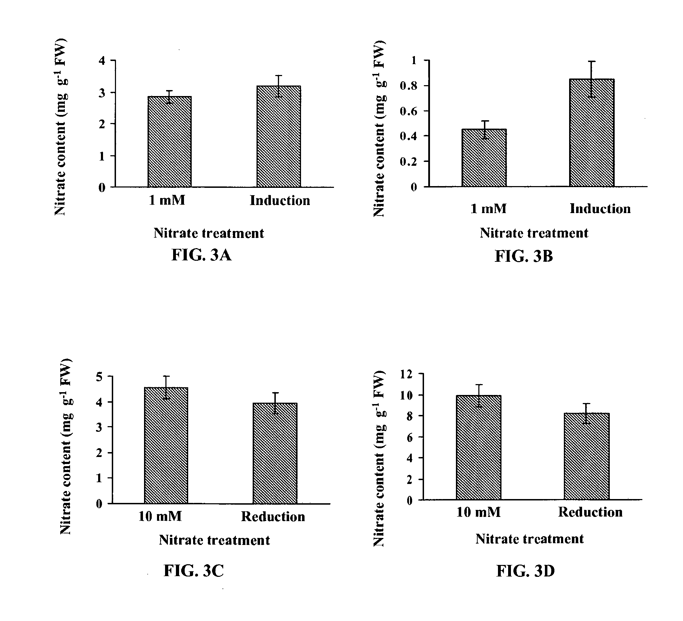

After a 2 hour nitrogen induction, nitrate levels in the shoots increased from 2.85 mg/gFW to 3.2 mg/gFW, an 11% change (Figure 3A), and no significant difference in the expression levels of various genes was detected. In the roots nitrate levels increased from 0.45 mg/gFW to 0.85 mg/gFW after a 2 hour nitrogen induction, approximately a 90% change (Figure 3B), and 295 significant genes were identified (273 up-regulated, and 22 down-regulated) (Table 2).

After a 2 hour nitrogen reduction, nitrate levels in the shoots decreased from 4.55 mg/gFW to 3.95 mg/gFW, a 12% change (Figure 3C), and no significant difference in the expression levels of various genes was detected. Nitrate levels in the roots decreased from 9.9 mg/gFW to 8.2 mg/gFW after a 2 hour nitrogen reduction, an 18% change (Figure 3D), and 401 significant genes were identified (339 up-regulated, and 62 down-regulated) (Table 2).

Among the 295 genes responsive to nitrogen induction and the 401 genes responsive to nitrogen reduction, 170 genes were overlapping, 125 genes responded specifically to nitrogen induction and 231 genes responded specifically to nitrogen reduction. Of the 125 genes that responded to nitrogen induction, 107 were up-regulated, and 18 down-regulated. Of the 231 genes that responded specifically to nitrogen reduction, 174 were up-regulated, and 57 down-regulated.

### Example 9. Generation And Selection Of Transgenic Rice Plants Over-Expressing NUE Candidate Genes

The genes that were identified in Examples 7 and 8 as being up-regulated or down-regulated were functionally classified and approximately 50 genes were selected that responded to either stable low nitrogen concentration, or to nitrogen induction, or to nitrogen reduction, or to both induction and reduction as NUE candidate genes. Most of the genes selected belong to three major functional groups: nitrogen metabolism, carbon metabolism, and those with putative regulatory functions (transcription factors and protein kinases). Full length cDNAs of all the selected genes were amplified and cloned into a binary vector. Transgenic rice plants were generated through Agrobacterium-mediated transformation of a transformable "Javanica" *Oryza sativa* which belongs to the Japonica species to constitutively over-express these NUE genes. Five to 10 independent events were generated for each construct. Transgene expression levels were tested by semi-quantitative RT-PCR, using *TUBULIN* as an internal control.

Transgenic plants were grown in a mixture of peat moss and vermiculite (1:4) (SunGro Horticulture Canada Ltd. BC, Canada) with the addition of a nutrient solution with different amount of nitrate once a week. The nutrient solution contained 4 mM MgSO₄, 5 mM KCl, 5 mM CaCl₂, 1 mM KH₂PO₄, 0.1 mM Fe-EDTA, 0.5 mM MES (pH6.0), 9 µ M MnSO₄, 0.7 µM Zn SO₄, 0.3 µM CuSO₄, 46 µM NaB₄O₇ and 0.2 µM (NH₄)₆Mo₇O₂. To screen at the vegetative stage, low nitrogen concentration (1 mM nitrate) was used once a week for four weeks. To screen at the end of the reproductive stage, a low-medium nitrogen concentration (3mM nitrate) was used once a week till harvest. Plants were grown in a growth room with 16 hr light (approximately 400 µmolm⁻²s⁻¹) at 28-30°C and 8 hr dark at 22-24°C for the first four weeks, then one week short-day treatment (10 hr light/14 hr dark) to promote flower, and then back to long-day till harvest.

T1 generation plants were screened by taking 5 transgenic events per construct and approximately 16 plants per event. Phosphomannose isomerase (PMI) tests were used for genotyping to detect the selectable marker PMI (Negrotto D, et al., Plant Cell Reports, 2000, 19: 798-803; Reed J, et al., In Vitro Cellular & Developmental Biology-Plant 2001, 37: 127-132; Wright M, et al., Plant Cell Reports, 2001, 20: 429-436). Briefly, leaf samples were grounded in 300 µl TraitChek sample buffer (Strategic Diagnostics Inc., Delaware, USA) and placed in an eppendorf tube. One SeedChek PMI test strip (Strategic Diagnostics Inc. Part, Delaware, USA) was inserted into the eppendorf tube for approximately 15 minutes by which time the marker lines on the test strip were clear. The appearance of one red line (control) on the strip indicates a negative result. The appearance of two red lines (control and test) on the strip indicates a positive result. The data from PMI positive plants was averaged to compare with the data from PMI negative plants as well as the wild type control plants.

Phenotype screening of T1 generation plants involved assessing NUE. NUE can be divided into two parts, one being assimilation efficiency mostly occurring at the vegetative stage; while the other being utilization efficiency mostly involving recycling of nitrogen at the reproductive stage (Good AG, et al., Trends Plant Sci., 2004, 9: 597-605). To screen plants at the vegetative stage, plants were grown under low nitrogen concentration (1 mM nitrate) for 4 weeks to examine the growth rate, shoot and root biomass of transgenic lines and wild type control plants. To screen plants at the reproductive stage, plants were grown under a low-medium nitrogen concentration (3mM nitrate) until the end of the reproductive stage to assess tiller and panicle number, shoot biomass and seed yield. Constructs with at least two events from the T1 generation showing improved NUE phenotype were selected for further analysis in T2 generation. Approximately 6% of such constructs were selected for further analysis from each type of the screening; none overlapping. Of these, approximately 50% showed the same phenotype in T2 generation as they had shown in the T1 generation.

### Example 10. Transgenic Rice Over-Expressing An Early Nodulin Gene (LOC_Os06g05010), Showed Increased Panicle Number, Shoot Dry Biomass And Seed Yield Under Limiting Nitrogen Condition

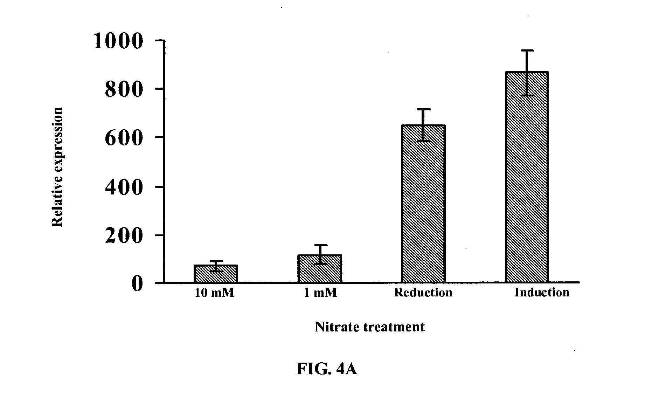

A transgenic rice over-expressing an early nodulin gene (LOC_Os06g05010) was generated and selected as set forth in Example 9. The early nodulin gene responded to either nitrogen induction or nitrogen reduction in a microarray expression profiling experiment, as described in Example 7 (Fig. 4a). All the five events from the T1 generation of this transgenic rice showed higher seed yield in PMI positive plants compared to PMI negative plants. Four T2 generation lines (from two different events) were tested again for NUE under both vegetative and reproductive stage growing conditions as described in Example 9 (a total of 10 plants for each T2 generation line were analyzed and results presented are mean values days after sowing). The transgenic rice plants did not show a significant difference at the vegetative stage compared to wild type rice plants. The transgenic plants appeared to grow slightly slower than wild type plants at 21 days after sowing, but appeared to grow quicker at 35 days and had slightly higher leaf number and tiller number than wild type plants at 42 days after sowing (Table 2). At the end of the reproductive stage the transgenic plants had similar tiller number, but increased shoot dry biomass, increased number of spikes and spikelets, and increased seed yield per plant compared to wild type (Table 3). These progeny plants were subjected to do a T3 generation test on both limiting nitrogen concentration (3 mM) and high nitrogen concentration (10 mM) conditions. The same trend was observed from these T3 plants, that the transgenic plants had increased shoot dry biomass, number of spikes and spikelets, and seed yield compared to wild type, not only under the limiting nitrogen concentration, but also under the high nitrogen concentration (Table 4) (data are mean ± SD (n = 10 plants)).

**Table 2**

| | Leaf Number | | | | Tiller Number | | | |
|---|---|---|---|---|---|---|---|---|
| | 21 Days | 28 Days | 35 Days | 42 Days | 21 Days | 28 Days | 35 Days | 42 Days |
| OX-02#3 | 15.8 | 20 | 24.8 | 26.8 | 3 | 4.5 | 5.3 | 6 |
| OX-02#15 | 12.7 | 17.5 | 23.2 | 25.6 | 4 | 4 | 5 | 5.5 |
| OX-03#2 | 13.5 | 19 | 23.2 | 26.4 | 3.2 | 4.2 | 5.4 | 5.8 |
| OX-03#3 | 13 | 19.5 | 22.4 | 25.5 | 3.1 | 4.3 | 5.4 | 5.7 |
| Wild Type | 16 | 20 | 22.2 | 25.3 | 3.5 | 4.5 | 5.2 | 5.4 |

**Table 3**

| | Total tillers | Shoot dry weight (g) | Spikes | Spikelets | Seed Yield (g) |
|---|---|---|---|---|---|
| OX-02#3 | 6.8 | 9.9 | 5 | 510 | 8.9 |
| OX-02#15 | 7 | 9.9 | 4.7 | 502 | 8.6 |
| OX-03#2 | 6.9 | 10 | 5 | 484 | 8.8 |
| OX-03#3 | 7 | 10.1 | 4.9 | 476 | 8.7 |
| Wild Type | 6.9 | 9.1 | 4 | 403 | 7.9 |

**Table 4**

| | Total tillers | Shoot dry weight (g) | Spikes | Spikelets | Seed Yield (g) |
|---|---|---|---|---|---|
| 3 mM Nitrate | | | | | |
| OX-1 | 5.5 ± 0.4 | 5.1 ± 0.6 | 3.2 ± 0.2 | 203 ± 23 | 3.8 ± 0.4 |
| OX-2 | 5.4 ± 0.4 | 4.9 ± 0.5 | 3.1 ± 0.3 | 197 ± 18 | 3.7 ± 0.3 |
| Wild Type | 5.2 ± 0.5 | 4.4 ± 0.4 | 2.7 ± 0.2 | 173 ± 18 | 3.1 ± 0.4 |

| 10 mM Nitrate | | | | | |
|---|---|---|---|---|---|
| OX-1 | 7.0 ± 0.6 | 10 ± 0.9 | 4.8 ± 0.4 | 465 ± 41 | 8.7 ± 0.9 |
| OX-2 | 6.9 ± 0.5 | 9.8 ± 1.0 | 4.9 ± 0.5 | 475 ± 44 | 8.8 ± 0.8 |
| Wild Type | 6.7 ± 0.6 | 8.9 ± 1.1 | 4.1 ± 0.5 | 395 ± 32 | 7.7 ± 0.8 |

### Example 11. The Early Nodulin Gene (LOC_Os06g05010) (Osenod93) Encodes A Protein That Is Expressed In Roots At A Higher Level Than In Shoots And Responds To Nitrogen Concentration

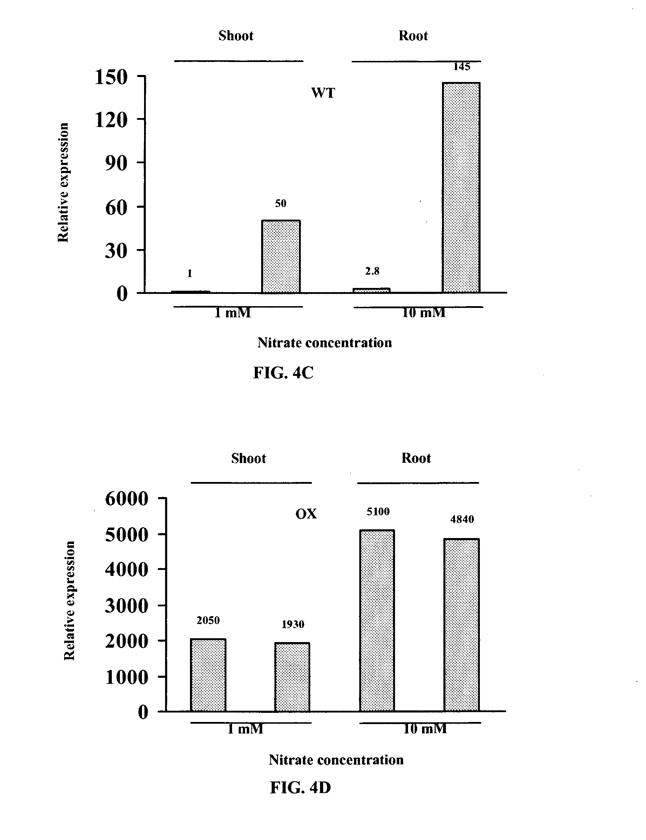

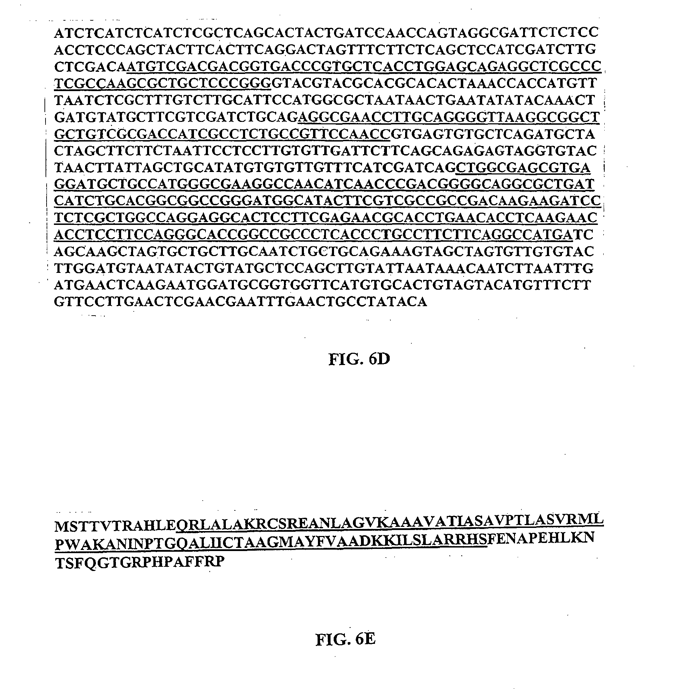

The early nodulin gene (LOC_Os06g05010) and other genes showing sequence similarity in rice has been designated as early nodulin 93, putative expressed (TIGR rice genome annotation data base). In the rice genome, there are 5 more early nodulin 93 genes located in the same region, with similar molecular weight and pI, and all expected to be localized in mitochondria except *Os06g04940* (Table 5). This early nodulin gene (LOC_Os06g05010) (*OsENOD93*) consists of three exons and two introns and encodes a protein of 116 amino acids (Figures 6C, 6D, and 6E) with a molecular weight 12424 Da and a pI of 10.95 (Table 5). Expression of this early nodulin gene in different plant parts at various growth stages was determined as described in Example 7. The expression profiling in different plant parts at various growth stages shows that this early nodulin gene is expressed at high level in roots specifically at panicle emergence stage (Figure 4B). Also, the expression level of this early nodulin gene in roots of 4 week old wild type plants was 50 times higher than in shoots (Figure 4C). Transgenic plants that constitutively overexpress this early nodulin gene had transcript levels several fold higher than wild type, with shoots and roots having almost similar transcript levels (Figure 4D). Plants grown at higher nitrogen concentrations showed increased expression of this early nodulin gene compared to plants grown at lower nitrogen concentrations (Figures 4C and 4D).

The expression of the early nodulin gene under different nitrogen conditions was examined at 30 and 60 days after sowing by quantitative real-time PCR as described in Example 7, with the gene expression at each nitrate treatment normalized to the wild type shoot grown at 1 mM nitrate 30 days after sowing. The results show that the early nodulin gene in wild-type plants is expressed in the root at higher levels than in other parts of the plant and that in both wild type and transgenic plants the expression level of the early nodulin gene increases with increasing nitrogen concentration (Table 6). Although the transgenic plants had high level of expression in leaves, N status in shoots were similar between wild type and transgenic plants (data not shown), suggesting that other partners in shoots are probably needed to fulfill the function of OsENOD93.

**Table 5**

| Gene | Protein Similarity | Amino Acids | Molecular Mass | pI | Localization^{a} | ENOD93 Domain^{b} |
|---|---|---|---|---|---|---|
| Os06g05010 | ENOD93 | 116 | 12424 | 10.95 | Mitochondria | 12....90 |
| Os06g04990 | ENOD93 | 115 | 12307 | 10.95 | Mitochondria | 11....89 |
| Os06g05020 | ENOD93 | 115 | 12277 | 10.95 | Mitochondria | 11....89 |
| Os06g04950 | ENOD93 | 115 | 12233 | 10.29 | Mitochondria | 11....89 |
| Os06g05000 | ENOD93 | 115 | 12167 | 10.35 | Mitochondria | 11....89 |
| Os06g04940 | ENOD93 | 139 | 14897 | 9.98 | Chloroplast | 3....113 |

| | | | | | | |
|---|---|---|---|---|---|---|
| ^{a}PSORT (Horton et al., *Nucleic Acids Res.,* 2007, 35:W585-W587)/TargetP1.1 (Emanuelsson et al., J. Mol. Biol., 2000, 300:1005-16). ^{b}Pfam 22.0 (Finn et al., Nucleic Acids Res., 2008, Database Issue 36:D281-D288) | | | | | | |

**Table 6**

| | 30 Days after sowing | | 60 Days after sowing | | | | |
|---|---|---|---|---|---|---|---|
| | Leaf | Root | Inflorescence | Flag Leaf | Leaf | Stem | Root |
| 1 mM Nitrate | | | | | | | |
| WT | 1 | 50 | 5.7 | 3.8 | 1.6 | 8.2 | 105 |
| OX^{a} | 2050 | 1245 | 292 | 2496 | 3674 | 395 | 2648 |
| 3 mM Nitrate | | | | | | | |
| WT | 1.5 | 92 | 9.5 | 6.3 | 2.7 | 15.6 | 218 |
| OX^{a} | 3640 | 2217 | 368 | 4125 | 5163 | 504 | 3441 |
| 10 mM Nitrate | | | | | | | |
| WT | 2.8 | 145 | 21 | 9.7 | 3.9 | 34.4 | 342 |
| OX^{a} | 5100 | 3142 | 456 | 6184 | 6842 | 595 | 4217 |

| | | | | | | | |
|---|---|---|---|---|---|---|---|
| ^{a}transgenic plant over-expressing early nodulin gene | | | | | | | |

### Example 12. The Roots Of Transgenic Plants Over-Expressing The Early Nodulin Gene (LOC_Os06g05010) Have Increased Amino Acid Content And Total Nitrogen

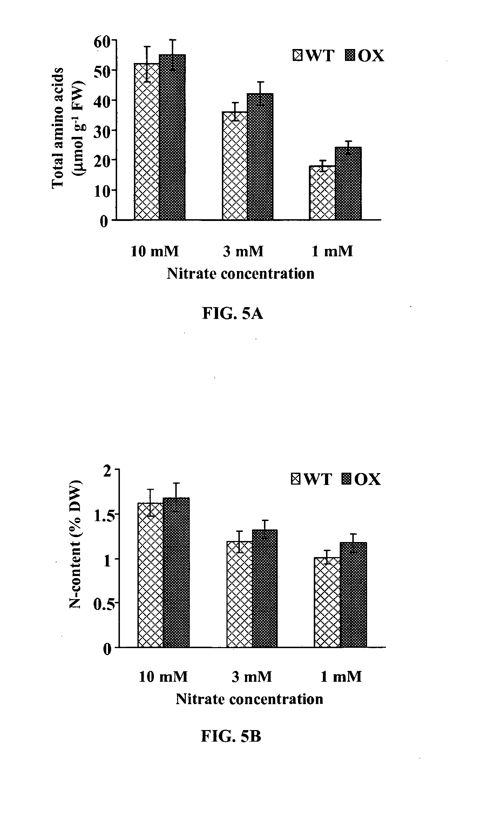

Transgenic plants over-expressing the early nodulin gene (LOC_Os06g05010) (*OsENOD93*) were grown as described in Example 9 and the amino acid content and total nitrogen content of the roots and shoots was determined as described in Examples 4 and 6. The roots of transgenic plants over-expressing the early nodulin gene (LOC_Os06g05010) (*OsENOD93*) had higher total amino acids (Figure 5A) and total nitrogen content (Figure 5B) than wild type plants, specifically when nitrogen was limiting. The amino acids and nitrogen content in shoots were similar between wild type and transgenic plants. Nitrate content in both shoots and roots were similar in transgenic and wild type plants.

### Example 13. The Increased Total Amino Acid And Total Nitrogen Content Seen At 30 Days After Sowing (DAS) Was More Pronounced At 60 DAS

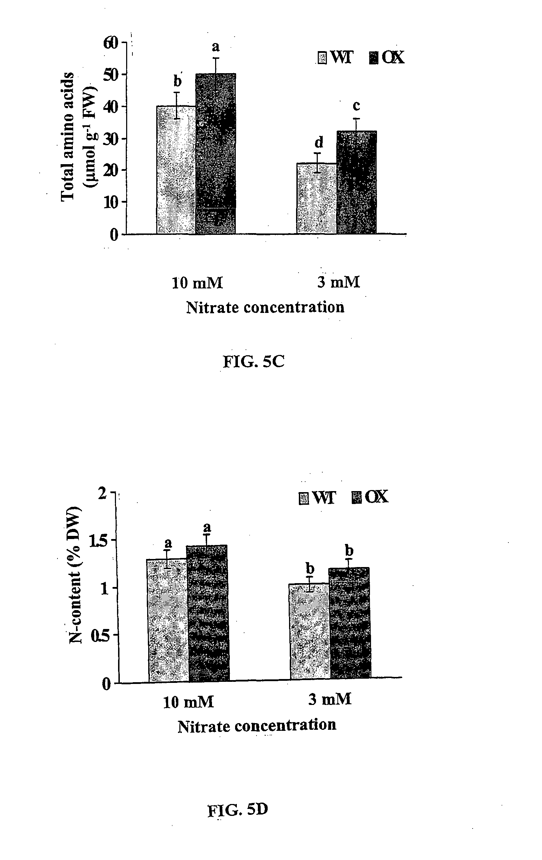

Transgenic plants over-expressing the early nodulin gene (LOC_Os06g5010) (*OsENOD93*) were grown as described in Example 9 and the total amino acid content and total nitrogen content of the roots was determined as described in Examples 4 and 6. The roots of transgenic plants over-expressing the early nodulin gene (LOC_Os06g05010) (*OsENOD93*) had higher total amino acids (Figure 5C) and total nitrogen content (Figure 5D) than wild type plants, specifically when nitrogen was limiting. This higher total amino acids and total nitrogen was more pronounced at 60DAS (Figures 5C and 5D) than at 30DAS (Figures 5A and 5B).

### Example 14. Transgenic Rice Plants Over-Expressing The OsENOD93 Gene Showed Increased Amino Acids In The Roots And Xylem Sap Under Limiting Nitrogen Conditions

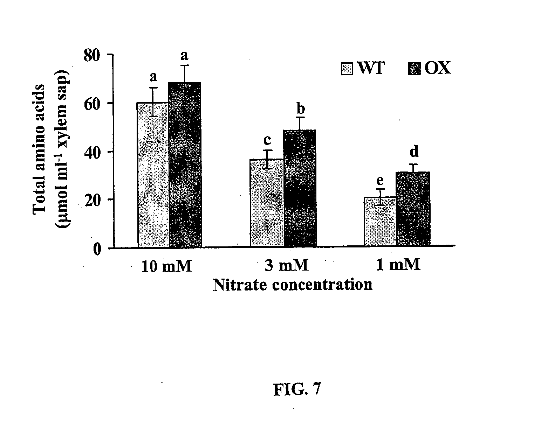

Transgenic rice plants were generated and selected through *Agrobacterum*-mediated transformation as described in Example 9. Because *OsENOD93* expression was high, and because amino acid accumulation was higher in transgenic roots, the amino acid content of the xylem sap was determined. Xylem sap was collected according to Shi, et al., (Shi, et al., The Plant Cell, 2002, 14: 465-477. The sap was diluted in distilled water and total amino acids were assayed according to Rosen (Rosen, Biochemistry and Biophysics, 1957, 67: 10-15). Transgenic plants over-expressing the *OsENOD93* gene were determined to have approximately 15% higher total amino acids in the xylem sap as compared to wild-type, specifically when nitrogen was limiting. (See Figure 7).

### Example 15. The Early Nodulin Gene (OsENOD93) Encodes A Transcript That Is Expressed In Vascular Bundles, Epidermis, And Endodermis

To further characterize the expression of *OsENOD93,* its transcript abundance was analyzed via *in situ* hybridization. For in situ hybridization analysis, samples were taken from 4 week old wild-type rice roots. These samples were cut into .5 to 1.0 cm sections and fixed in 4% formaldehyde. The tissues were then dehydrated and embedded in paraffin. The eight micrometer thick tissue sections were cut using a microtome and mounted on microscope slides coated with poly-lysine (Biolabs). Following treatment with xylene to remove paraffin, the sections were treated for thirty minutes with proteinase K (10 micrograms/milliliter). *In situ* hybridization was performed as described previously (Komminoth, Diagnostic Molecular Pathology, 1992, 1: 142-150), using digoxigenin (DIG)-labeled sense and antisense *OsENOD93-*specific RNA probes (Roche Diagnostics, Penzenberg, Germany). The sections were photographed using a DMIR2 microscope (Leica, Germany) and a DC500 camera (Leica, Germany). Expression, as measured by the presence of transcripts, was detected in the vascular bundles, epidermis, and endodermis. The expression pattern of *OsENOD93* suggests a role in the transport of compounds, including from root to shoot.

### Example 16. Generation And Selection Of Transgenic Onion Expressing The OsENOD Protein Demonstrates

That *OsENOD93* Co-Localizes With A Known Mitochondrial Marker ProteinTo better understand the subcellular localization of OsENOD93 protein, a polynucleotide encoding *OsENOD93* was amplified by PCR and cloned into a C-terminal Yellow Fluorescent Protein (YFP) reporter gene fusion gateway-compatible vector pEarlyGate 101 (Earley, et al., The Plant Journal, 2006, 45: 616-629). The *OsENOD93-*YFP fusion protein and control β-ATPase-RFP (Genbank Accession No. P17614) constructs were biolistically transformed into onion epidermal cells. Images of the fluorescent protein-containing cells were taken twenty hours post-bombardment through epiflourescence microscopy. After a sufficient length of time for gene expression and cell sorting, images taken confirmed a clear colocalization of *OsENOD93* with β-ATPase-RFP, a known mitochondrial marker protein. Protein colocalizations, if present, appeared as an orange signal due to the overlay of the two fluorophores (red and green) in the same cell. As shown in Table 5, the rice genome contains five such *ENOD93* genes located in the same chromosomal region. These rice ENOD93 genes have similar molecular weights, similar pIs, are predicted to contain two transmembrane domains, and are expected to be localized to the mitochondrial membrane.

## Claims

1. A transgenic plant, a transgenic plant seed or a transgenic plant cell, comprising a recombinant nucleic acid encoding a functional OsENOD93 protein, wherein the recombinant nucleic acid comprises:
(a) a nucleotide sequence set forth as SEQ ID NO: 1;
(b) a nucleic acid that specifically hybridizes to the complement of SEQ ID NO: 1 under stringent hybridization conditions;
(c) a nucleotide sequence set forth as SEQ ID NO: 2;
(d) a nucleic acid that specifically hybridizes to the complement of SEQ ID NO: 2 under stringent hybridization conditions; or
(e) a nucleotide sequence encoding a OsENOD93 protein comprising an amino acid sequence set forth as SEQ ID NO: 3.

2. The transgenic plant, transgenic plant seed or transgenic plant cell of claim 1, wherein the transgenic plant, transgenic plant seed or transgenic plant cell is a monocot.

3. The transgenic plant, transgenic plant seed or transgenic plant cell of claim 2, wherein the transgenic plant, transgenic plant seed or transgenic plant cell is maize.

4. The transgenic plant, transgenic plant seed or transgenic plant cell of claim 2, wherein the transgenic plant, transgenic plant seed or transgenic plant cell is rice.

5. The transgenic plant, transgenic plant seed or transgenic plant cell of claim 1, wherein the transgenic plant, transgenic plant seed or transgenic plant cell is a dicot.

6. A method of producing a transgenic plant cell, comprising:
introducing a recombinant nucleic acid encoding a functional OsENOD93 protein into a plant cell to produce the transgenic plant cell, wherein the recombinant nucleic acid comprises:
(a) a nucleotide sequence set forth as SEQ ID NO: 1;
(b) a nucleic acid that specifically hybridizes to the complement of SEQ ID NO: 1 under stringent hybridization conditions;
(c) a nucleotide sequence set forth as SEQ ID NO: 2;
(d) a nucleic acid that specifically hybridizes to the complement of SEQ ID NO: 2 under stringent hybridization conditions; or
(e) a nucleotide sequence encoding a OsENOD93 protein comprising an amino acid sequence set forth as SEQ ID NO: 3.

7. A method of producing a transgenic plant, comprising:
producing a transgenic plant cell in accordance with the method of Claim 6; and
regenerating the transgenic plant from the transgenic plant cell.

8. A method of increasing or improving nitrogen use efficiency, nitrate levels, lateral root growth, seed yield, stress tolerance, amino acid levels and/or biomass in a transgenic plant, a transgenic plant seed or a transgenic plant cell, comprising:
expressing in the transgenic plant, transgenic plant seed or transgenic plant cell a recombinant nucleic acid encoding a functional OsENOD93, wherein the recombinant nucleic acid comprises:
(a) a nucleotide sequence set forth as SEQ ID NO: 1;
(b) a nucleic acid that specifically hybridizes to the complement of SEQ ID NO: 1 under stringent hybridization conditions;
(c) a nucleotide sequence set forth as SEQ ID NO: 2;
(d) a nucleic acid that specifically hybridizes to the complement of SEQ ID NO: 2 under stringent hybridization conditions; or
(e) a nucleotide sequence encoding a OsENOD93 protein comprising an amino acid sequence set forth as SEQ ID NO: 3.

9. The method of any one of claims 6 to 8, wherein the recombinant nucleic acid is constitutively expressed in the transgenic plant, transgenic plant seed or transgenic plant cell.

10. The method of claim 8 or claim 9, wherein the transgenic plant, transgenic plant seed or transgenic plant cell shows an increase in seed yield, amino acid levels and/or biomass.

11. The method of claim 10, wherein the transgenic plant, transgenic plant seed or transgenic plant cell shows increased nitrogen use efficiency.

12. The method of any one of claims 6 to 11, wherein the transgenic plant, transgenic plant seed or transgenic plant cell is a monocot.

13. The method of claim 12, wherein the transgenic plant, transgenic plant seed or transgenic plant cell is maize.

14. The method of claim 12, wherein the transgenic plant, transgenic plant seed or transgenic plant cell is rice.

15. The method of any one of claims 6 to 11, wherein the transgenic plant, transgenic plant seed or transgenic plant cell is a dicot.

## Patentansprüche

1. Transgene Pflanze, transgener Pflanzensamen oder transgene Pflanzenzelle, umfassend eine rekombinante Nukleinsäure, die ein funktionelles OsENOD93-Protein kodiert, wobei die rekombinante Nukleinsäure Folgendes beinhaltet:
(a) eine Nukleotidsequenz gemäß SEQ ID NR. 1;
(b) eine Nukleinsäure, die unter stringenten Hybridisierungsbedingungen speziell an das Komplement von SEQ ID NR. 1 hybridisiert;
(c) eine Nukleotidsequenz gemäß SEQ ID NR. 2;
(d) eine Nukleinsäure, die unter stringenten Hybridisierungsbedingungen speziell an das Komplement von SEQ ID NR. 2 hybridisiert; oder
(e) eine Nukleotidsequenz, die ein OsENOD93-Protein kodiert, das eine Aminosäure gemäß SEQ ID NR. 3 umfasst.

2. Transgene Pflanze, transgener Pflanzensamen oder transgene Pflanzenzelle nach Anspruch 1, wobei die transgene Pflanze, der transgene Pflanzensamen oder die transgene Pflanzenzelle eine Monokotyledone ist.

3. Transgene Pflanze, transgener Pflanzensamen oder transgene Pflanzenzelle nach Anspruch 2, wobei die transgene Pflanze, der transgene Pflanzensamen oder die transgene Pflanzenzelle Mais ist.

4. Transgene Pflanze, transgener Pflanzensamen oder transgene Pflanzenzelle nach Anspruch 2, wobei die transgene Pflanze, der transgene Pflanzensamen oder die transgene Pflanzenzelle Reis ist.

5. Transgene Pflanze, transgener Pflanzensamen oder transgene Pflanzenzelle nach Anspruch 1, wobei die transgene Pflanze, der transgene Pflanzensamen oder die transgene Pflanzenzelle eine Dikotyledone ist.

6. Verfahren zum Erzeugen einer transgenen Pflanzenzelle, das Folgendes beinhaltet:
Einführen einer rekombinanten Nukleinsäure, die ein funktionelles OsENOD93-Protein kodiert, in eine Pflanzenzelle zum Erzeugen der transgenen Pflanzenzelle, wobei die rekombinante Nukleinsäure Folgendes beinhaltet:
(a) eine Nukleotidsequenz gemäß SEQ ID NR. 1;
(b) eine Nukleinsäure, die unter stringenten Hybridisierungsbedingungen speziell an das Komplement von SEQ ID NR. 1 hybridisiert;
(c) eine Nukleotidsequenz gemäß SEQ ID NR. 2;
(d) eine Nukleinsäure, die unter stringenten Hybridisierungsbedingungen speziell an das Komplement von SEQ ID NR. 2 hybridisiert; oder
(e) eine Nukleotidsequenz, die ein OsENOD93-Protein kodiert, das eine Aminosäuresequenz gemäß SEQ ID NR. 3 beinhaltet.

7. Verfahren zum Produzieren einer transgenen Pflanze, das Folgendes beinhaltet:
Produzieren einer transgenen Pflanzenzelle gemäß dem Verfahren von Anspruch 6; und
Regenerieren der transgenen Pflanze von der transgenen Pflanzenzelle.

8. Verfahren zum Erhöhen oder Verbessern der Stickstoffnutzungseffizienz, des Nitratgehalts, des lateralen Wurzelwachstums, des Samenertrags, der Belastungstoleranz, des Aminosäuregehalts und/oder der Biomasse in einer transgenen Pflanze, einem transgenen Pflanzensamen oder einer transgenen Pflanzenzelle, das Folgendes beinhaltet:
Exprimieren einer rekombinanten Nukleinsäure, die ein funktionelles OsENOD93 kodiert, in der transgenen Pflanze, dem transgenen Pflanzensamen oder der transgenen Pflanzenzelle, wobei die rekombinante Nukleinsäure Folgendes beinhaltet:
(a) eine Nukleotidsequenz gemäß SEQ ID NR. 1;
(b) eine Nukleinsäure, die unter stringenten Hybridisierungsbedingungen speziell an das Komplement von SEQ ID NR: 1 hybridisiert;
(c) eine Nukleotidsequenz gemäß SEQ ID NR. 2;
(d) eine Nukleinsäure, die unter stringenten Hybridisierungsbedingungen speziell an das Komplement von SEQ ID NR. 2 hybridisiert; oder
(e) eine Nukleotidsequenz, die ein OsENOD93-Protein kodiert, das eine Aminosäuresequenz gemäß SEQ ID NR. 3 beinhaltet.

9. Verfahren nach einem der Ansprüche 6 bis 8, wobei die rekombinante Nukleinsäure konstitutiv in der transgenen Pflanze, dem transgenen Pflanzensamen oder der transgenen Pflanzenzelle exprimiert wird.

10. Verfahren nach Anspruch 8 oder Anspruch 9, wobei die transgene Pflanze, der transgene Pflanzensamen oder die transgene Pflanzenzelle eine Zunahme an Samenertrag, Aminosäuregehalt und/oder Biomasse zeigt.

11. Verfahren nach Anspruch 10, wobei die transgene Pflanze, der transgene Pflanzensamen oder die transgene Pflanzenzelle eine erhöhte Stickstoffnutzungseffizienz zeigt.

12. Verfahren nach einem der Ansprüche 6 bis 11, wobei die transgene Pflanze, der transgene Pflanzensamen oder die transgene Pflanzenzelle eine Monokotyledone ist.

13. Verfahren nach Anspruch 12, wobei die transgene Pflanze, der transgene Pflanzensamen oder die transgene Pflanzenzelle Mais ist.

14. Verfahren nach Anspruch 12, wobei die transgene Pflanze, der transgene Pflanzensamen oder die transgene Pflanzenzelle Reis ist.

15. Verfahren nach einem der Ansprüche 6 bis 11, wobei die transgene Pflanze, der transgene Pflanzensamen oder die transgene Pflanzenzelle eine Dikotyledone ist.

## Revendications

1. Plante transgénique, graine de plante transgénique ou cellule de plante transgénique, comprenant un acide nucléique recombiné codant pour une protéine fonctionnelle OsENOD93, l'acide nucléique recombiné comprenant :
(a) une séquence nucléotidique, laquelle est la SÉQ. ID n° 1 ;
(b) un acide nucléique qui s'hybride spécifiquement avec le complément de la SÉQ. ID n° 1 dans des conditions rigoureuses d'hybridation ;
(c) une séquence nucléotidique, laquelle est la SÉQ. ID n° 2 ;
(d) un acide nucléique qui s'hybride spécifiquement avec le complément de la SÉQ. ID n° 2 dans des conditions rigoureuses d'hybridation ; ou
(e) une séquence nucléotidique codant pour une protéine OsENOD93 comprenant une séquence d'acides aminés, laquelle est la SÉQ. ID n° 3.

2. Plante transgénique, graine de plante transgénique ou cellule de plante transgénique selon la revendication 1, la plante transgénique, la graine de plante transgénique ou la cellule de plante transgénique étant une plante, une graine ou une cellule monocotylédone.

3. Plante transgénique, graine de plante transgénique ou cellule de plante transgénique selon la revendication 2, la plante transgénique, la graine de plante transgénique ou la cellule de plante transgénique étant un plant, une graine ou une cellule de maïs.

4. Plante transgénique, graine de plante transgénique ou cellule de plante transgénique selon la revendication 2, la plante transgénique, la graine de plante transgénique ou la cellule de plante transgénique étant un plant, une graine ou une cellule de riz.

5. Plante transgénique, graine de plante transgénique ou cellule de plante transgénique selon la revendication 1, la plante transgénique, la graine de plante transgénique ou la cellule de plante transgénique étant une plante, une graine ou une cellule dicotylédone.

6. Procédé de production d'une cellule de plante transgénique, consistant à :
introduire un acide nucléique recombiné codant pour une protéine fonctionnelle OsENOD93 dans une cellule végétale pour produire la cellule de plante transgénique, l'acide nucléique recombiné comprenant :
(a) une séquence nucléotidique, laquelle est la SÉQ. ID n° 1 ;
(b) un acide nucléique qui s'hybride spécifiquement avec le complément de la SÉQ. ID n° 1 dans des conditions rigoureuses d'hybridation ;
(c) une séquence nucléotidique, laquelle est la SÉQ. ID n° 2 ;
(d) un acide nucléique qui s'hybride spécifiquement avec le complément de la SÉQ. ID n° 2 dans des conditions rigoureuses d'hybridation ; ou
(e) une séquence nucléotidique codant pour une protéine OsENOD93 comprenant une séquence d'acides aminés, laquelle est la SÉQ. ID n° 3.

7. Procédé de production d'une plante transgénique, consistant à :
produire une cellule de plante transgénique par la méthode selon la revendication 6 ; et
régénérer la plante transgénique à partir de la cellule de plante transgénique.

8. Procédé d'augmentation ou d'amélioration du rendement d'utilisation de l'azote, des niveaux de nitrates, de la croissance des racines latérales, du rendement en graines, de la tolérance au stress, des niveaux d'acides aminés et/ou de la biomasse dans une plante transgénique, une graine de plante transgénique ou une cellule de plante transgénique, consistant à :
exprimer dans la plante transgénique, la graine de plante transgénique ou la cellule de plante transgénique un acide nucléique recombiné codant pour une protéine fonctionnelle OsENOD93, l'acide nucléique recombiné comprenant :
(a) une séquence nucléotidique, laquelle est la SÉQ. ID n° 1 ;
(b) un acide nucléique qui s'hybride spécifiquement avec le complément de la SÉQ. ID n° 1 dans des conditions rigoureuses d'hybridation ;
(c) une séquence nucléotidique, laquelle est la SÉQ. ID n° 2 ;
(d) un acide nucléique qui s'hybride spécifiquement avec le complément de la SÉQ. ID n° 2 dans des conditions rigoureuses d'hybridation ; ou
(e) une séquence nucléotidique codant pour une protéine OsENOD93 comprenant une séquence d'acides aminés, laquelle est la SÉQ. ID n° 3.

9. Procédé selon l'une quelconque des revendications 6 à 8, dans lequel l'acide nucléique recombiné est exprimé constitutivement dans la plante transgénique, la graine de plante transgénique ou la cellule de plante transgénique.

10. Procédé selon la revendication 8 ou la revendication 9, dans lequel la plante transgénique, la graine de plante transgénique ou la cellule de plante transgénique présente une augmentation du rendement en graines, des niveaux d'acides aminés et/ou de la biomasse.

11. Procédé selon la revendication 10, dans lequel la plante transgénique, la graine de plante transgénique ou la cellule de plante transgénique présente une augmentation du rendement d'utilisation de l'azote.

12. Procédé selon l'une quelconque des revendications 6 à 11, dans lequel la plante transgénique, la graine de plante transgénique ou la cellule de plante transgénique est une plante, une graine ou une cellule monocotylédone.

13. Procédé selon la revendication 12, dans lequel la plante transgénique, la graine de plante transgénique ou la cellule de plante transgénique est un plant, une graine ou une cellule de maïs.

14. Procédé selon la revendication 12, dans lequel la plante transgénique, la graine de plante transgénique ou la cellule de plante transgénique est un plant, une graine ou une cellule de riz.

15. Procédé selon l'une quelconque des revendications 6 à 11, dans lequel la plante transgénique, la graine de plante transgénique ou la cellule de plante transgénique est une plante, une graine ou une cellule dicotylédone.
